# EUROPÄISCHE PATENTSCHRIFT

(11) **EP 2 145 006 B1**
(45) Veröffentlichungstag und Bekanntmachung des Hinweises auf die Patenterteilung: **11.05.2011**
(21) Anmeldenummer: 08734603.7
(22) Anmeldetag: 12.03.2008
(51) Int. Cl.: C12N 15/75, C12N 9/04

(54) **EXPRESSIONSSYSTEM ZUR ANTIBIOTIKAFREIEN PRODUKTION VON POLYPEPTIDEN**
EXPRESSION SYSTEM USED FOR THE ANTIBIOTIC-FREE PRODUCTION OF POLYPEPTIDES
SYSTÈME D'EXPRESSION PERMETTANT LA PRODUCTION DE POLYPEPTIDES SANS ANTIBIOTIQUES

(30) Priorität: 04.05.2007 DE 102007021001
(43) Veröffentlichungstag der Anmeldung: 20.01.2010
(73) Patentinhaber: AB Enzymes GmbH, 64293 Darmstadt (DE)
(72) Erfinder: CADOT, Céline, 92370 Chaville (FR); PLOSS, Tina, 64331 Weiterstadt (DE); SCHWERDTFEGER, Ruth, 64291 Darmstadt (DE); WINTER, Bruno, 70190 Stuttgart (DE)
(74) Vertreter: Hiebl, Inge Elisabeth
(86) Internationale Anmeldenummer: PCT/EP2008/001977
(87) Internationale Veröffentlichungsnummer: WO 2008/135113

(56) Entgegenhaltungen:
- WO-A-2005/061716
- MORBIDONI H R ET AL: "Synthesis of sn-Glycerol 3 -Phosphate, a key precursor of membrane lipids, in Bacillus subtilis" JOURNAL OF BACTERIOLOGY, WASHINGTON, DC, US, Bd. 177, Nr. 20, 1. Oktober 1995 (1995-10-01), Seiten 5899-5905, XP002186763 ISSN: 0021-9193
- HAGG PETER ET AL: "A host/plasmid system that is not dependent on antibiotics and antibiotic resistance genes for stable plasmid maintenance in Escherichia coli" JOURNAL OF BIOTECHNOLOGY, ELSEVIER SCIENCE PUBLISHERS, AMSTERDAM, NL, Bd. 111, Nr. 1, 1. Juli 2004 (2004-07-01), Seiten 17-30, XP002316099 ISSN: 0168-1656
- CHEN XIAO HUA ET AL: "Comparative analysis of the complete genome sequence of the plant growth-promoting bacterium Bacillus amyloliquefaciens FZB42" NATURE BIOTECHNOLOGY, Bd. 25, Nr. 9, September 2007 (2007-09), Seiten 1007-1014, XP002493384 ISSN: 1087-0156

### SEQUENZPROTOKOLL

<110> AB Enzymes GmbH
<120> Expressionssystem zur antibiotikafreien Produktion von Polypeptiden
<130> 17327EP
<140> EP 08 734 603.7
   <141> 2008-03-12
<150> DE 10 2007 021 001.0
   <151> 2007-05-04
<160> 16
<170> Patent In version 3.3
<210> 1
   <211> 3715
   <212> DNA
   <213> Bacillus amyloliquefaciens
<220>
   <221> CDS
   <222> (1338)..(2372)
<400> 1
   aagggtacta tgggtaaacc tgtcgtagcc attgtcggaa gaccgaatgt gggaaaatcc      60
   acaatcttta accggattgc gggtgaaaga atttcaatag tagaagatac ccccggagtg     120
   acgcgggacc ggatatacag ttcggcggaa tggctgaatt atgattttaa cctgattgat     180
   acgggcggaa ttgatatcgg agacgagccg tttctgacac agatccgcca gcaggctgaa     240
   atcgccatgg acgaggctga tgtgatcatc tttatggtga acggccgcga aggtgtgacg     300
   tctgcggatg aagaagtggc aaaaatactg taccggacga aaaaaccggt cgtattagcc     360
   gttaataaat tagataatac cgaaatgaga gcgaacattt atgactttta tgcgctcggc     420
   tttggagaac cgtatccgat ttcggggaca cacggtttag gattgggaga tttgctcgat     480
   gcttgtgccg agcattttaa aaacattccg gagacgaagt acagtgatga tgtcgttcaa     540
   ttctgcctga tcggccgccc gaatgtcgga aaatcatccc ttgtcaatgc gatgctcggc     600
   gaagagcggg ttatcgtgag caacgtagcc ggaacgacta gagacgctgt ggatacggcg     660
   ttcacttaca atcagcagga atttgtaatc gttgacacgg cgggaatgag aaaaaagggt     720
   aaagtatatg aaacaacaga aaaatacagt gtgctgcggg cgttaaaagc cattgaccgc     780
   tcagacgtcg tcggcgttgt gctgaatgca gaagaaggca tccttgagca ggacaagcgg     840
   atcgccggat acgcccatga agccgggaaa gccgtcgtca ttatcgtaaa caaatgggat     900
   gccgttgata aggacgagcg cacgatgaaa gaatttgaac agaatattcg ggagcatttc     960
   caatttctcg attatgcgcc ggtgctgttt atgtcggcac tgacgacaaa gcggattcat    1020
   acactcatgc ctgcgattat taaagcgagt gaaaaccact ctctccgcgt gcagacaaat   1080
   atcttaaatg atgtcatcat ggatgcggtc gccatgaatc cgactccgac gcacaacggc   1140
   tcccgtctga aaatttatta tgcgactcaa gtcgctgtta agccgccgag cttcgttgtt   1200
   tttgtcaacg atccggaact gatgcatttt tcttatgaac gctttttaga aaaccgaatc   1260
   cgggacgctt tcggatttga aggtacgcca attaaaatat ttgcaagagc aagaaaataa   1320
   aaaggtgtga tacagag atg aaa aaa gtg gca atg ctt gga gcg gga agc      1370
                      Met Lys Lys Val Ala Met Leu Gly Ala Gly Ser
                      1               5                   10
   tgg ggc act gca ctt tct tta gtg ctg gct gat aac gga cat caa gtc     1418
   Trp Gly Thr Ala Leu Ser Leu Val Leu Ala Asp Asn Gly His Gln Val
               15                  20                  25
   atg atg tgg gga cac cgt gcc gaa ttg atc gat caa atc aac gaa ctg     1466
   Met Met Trp Gly His Arg Ala Glu Leu Ile Asp Gln Ile Asn Glu Leu
           30                  35                  40
   cat gaa aac aaa gat tac ctg ccg ggt gtg gag cta tca agt tct atc     1514
   His Glu Asn Lys Asp Tyr Leu Pro Gly Val Glu Leu Ser Ser Ser Ile
       45                  50                  55
   atc ggg aca gcc gat tta agc gag gct tta aaa gga gct gac ttc atc     1562
   Ile Gly Thr Ala Asp Leu Ser Glu Ala Leu Lys Gly Ala Asp Phe Ile
   60                  65                  70                  75
   att gtg gca gta ccg aca aaa gcc att cgg gaa gtg ctg aaa aag gct     1610
   Ile Val Ala Val Pro Thr Lys Ala Ile Arg Glu Val Leu Lys Lys Ala
                   80                  85                  90
   ctg ccg tac atc ccg aaa caa tcg att ttt gtt cat gtc agc aag gga     1658
   Leu Pro Tyr Ile Pro Lys Gln Ser Ile Phe Val His Val Ser Lys Gly
               95                  100                 105
   att gag ccg gat tcg ctt ctc cgc att tca gaa tta atg gag gag gag     1706
   Ile Glu Pro Asp Ser Leu Leu Arg Ile Ser Glu Leu Met Glu Glu Glu
           110                 115                 120
   ctg cct gag gag tac aga aaa gac atc gtc gtg ctt tca ggg ccg agc     1754
   Leu Pro Glu Glu Tyr Arg Lys Asp Ile Val Val Leu Ser Gly Pro Ser
       125                 130                 135
   cac gct gag gaa gtc gga tta aga cac ccg acg act gtt aca tca tct     1802
   His Ala Glu Glu Val Gly Leu Arg His Pro Thr Thr Val Thr Ser Ser
   140                 145                 150                 155
   tca aaa aat atc aag gct gca gaa gcg gtt cag gat tta ttc atg aac     1850
   Ser Lys Asn Ile Lys Ala Ala Glu Ala Val Gln Asp Leu Phe Met Asn
                   160                 165                 170
   cag cat ttc cgc gtc tat aca aat ccc gat atg atc ggt gtt gaa ata     1898
   Gln His Phe Arg Val Tyr Thr Asn Pro Asp Met Ile Gly Val Glu Ile
               175                 180                 185
   ggg gga gcg tta aaa aat atc atc gct ctt gca gcg ggg att aca gac     1946
   Gly Gly Ala Leu Lys Asn Ile Ile Ala Leu Ala Ala Gly Ile Thr Asp
           190                 195                 200
   gga ttg gga tac gga gat aat gca aaa gcg gcc tta atc acc cga ggt      1994
   Gly Leu Gly Tyr Gly Asp Asn Ala Lys Ala Ala Leu Ile Thr Arg Gly
       205                 210                 215
   ctt gct gaa atc gcc aga ctc ggc aca aag atg ggc gga aat ccg ctc      2042
   Leu Ala Glu Ile Ala Arg Leu Gly Thr Lys Met Gly Gly Asn Pro Leu
   220                 225                 230                 235
   acc ttt tcc ggc ctg acc ggc gta ggc gat tta atc gtg acg tgt aca      2090
   Thr Phe Ser Gly Leu Thr Gly Val Gly Asp Leu Ile Val Thr Cys Thr
                   240                 245                 250
   agc gtt cat tcc cga aac tgg cgc gcc ggc aac ctg ctc ggc aaa gga      2138
   Ser Val His Ser Arg Asn Trp Arg Ala Gly Asn Leu Leu Gly Lys Gly
               255                 260                 265
   tat aag ctg gaa gct gtc ctg gat aag atg ggg atg gtt gtt gaa ggc      2186
   Tyr Lys Leu Glu Ala Val Leu Asp Lys Met Gly Met Val Val Glu Gly
           270                 275                 280
   gtg cgg acg acg aaa gct gcg tat cag ctg tct caa aaa tat cag gtg      2234
   Val Arg Thr Thr Lys Ala Ala Tyr Gln Leu Ser Gln Lys Tyr Gln Val
       285                 290                 295
   aaa atg ccg atc aca gaa gcg ctt cac caa gta tta ttt aat ggg cag      2282
   Lys Met Pro Ile Thr Glu Ala Leu His Gln Val Leu Phe Asn Gly Gln
   300                 305                 310                 315
   aag gta gaa act gcc gta gaa tcc tta atg gcc aga gtg aaa acc cat      2330
   Lys Val Glu Thr Ala Val Glu Ser Leu Met Ala Arg Val Lys Thr His
                   320                 325                 330
   gag atg gaa gac ttg gtc aac aca ttc gaa aac cgg gtg aag              2372
   Glu Met Glu Asp Leu Val Asn Thr Phe Glu Asn Arg Val Lys
               335                 340                 345
   tgacaataac atgccgtcag catattctga agtgacgaaa gtacaaaacg cagaactctc   2432
   cggctgaaat cgcttaaaac atttgctgat ttaggcagaa gaggatgcag acgtacgcat   2492
   ctacgcatac tatagcatcg aagtccaaga gagtgtatct cagacgtaca ggtgaatata   2552
   gtcaacttga ctgaagcaaa gtccccctct ttgcttcagt ttttttgttt tttcaataga   2612
   tggaaaacct gttgatcttt tcaacatttg tatattaaaa tgaaatataa cgcttacaat   2672
   gatgggacgg ggggcacgaa cgtgagtgtg gcattgatga aaatgtggtt tgcgcttggt   2732
   gctatgggat taatgtttgt tgcggtcgcc tctatttatg tcagcaggta caaagtgaaa   2792
   aacaaactga taaaagcagc ggtttcttca ctcgcttatg cctgcatggt catctcggga   2852
   ttgatcgtgt taatggtcgt tttcagcggg cctgtcaatg aataaagcca aaagggggcg   2912
   cggaatgcat aagttaaaaa tggccgtcat aacggcaatg gcggtgcttc tgctgtcggg   2972
   ctgtctgtac cctgaagcaa aaaaaactga aaataaagta tcttacaaac atcagcttca   3032
   gcaggtgcaa gcggcagtgg atgaatttaa aaaggcgaac ggcggacttc tgccgattca   3092
   gacaaaagat atgaaaacac cgctctatca aaaatatccg atagatttta agcggctcgc   3152
   gcccagatac atcgaggagc cgccggcctc agcttatgaa agcggaggaa tgtaccaata   3212
   cgtgcttgtc gatgtggaaa ataagccgac cgtcaagctg gtcgatctcc aaatggcgga   3272
   agcaatccgc gacatgaagc tgcgtgtcaa aatgtatcag gaaaagcata catatcctcc   3332
   ctatgaggac gctgtttcaa aagggctgtt cactttaaat aaaaaaaagc tcggcatgaa   3392
   agactctcct tcagtcaaaa gtccggtttc aggcacgtct ctgccgcttt taatcggcgc   3452
   tgacggagaa atctatgccg actatcgcgt cgatctcgcc cgctgcctga aggaaaacaa   3512
   aaagaaaatc aaaccggggg cggaaattca ggatatttta tggaaagaga ctcctttcgt   3572
   cccggccttt tcagtcacat acaccgtaaa tgaaaaacag gaacccgttt ttttagaaag   3632
   tcaaacgaaa caggaatgaa cctttttccc gcgcatacaa atagggagaa aggttttttt   3692
   gattttgata gaaaagactg cct                                           3715
<210> 2
   <211> 345
   <212> PRT
   <213> Bacillus amyloliquefaciens
<400> 2
   Met Lys Lys Val Ala Met Leu Gly Ala Gly Ser Trp Gly Thr Ala Leu
   1               5                   10                  15
   Ser Leu Val Leu Ala Asp Asn Gly His Gln Val Met Met Trp Gly His
               20                  25                  30
   Arg Ala Glu Leu Ile Asp Gln Ile Asn Glu Leu His Glu Asn Lys Asp
           35                  40                  45
   Tyr Leu Pro Gly Val Glu Leu Ser Ser Ser Ile Ile Gly Thr Ala Asp
       50                  55                  60
   Leu Ser Glu Ala Leu Lys Gly Ala Asp Phe Ile Ile Val Ala Val Pro
   65                  70                  75                  80
   Thr Lys Ala Ile Arg Glu Val Leu Lys Lys Ala Leu Pro Tyr Ile Pro
                   85                  90                  95
   Lys Gln Ser Ile Phe Val His Val Ser Lys Gly Ile Glu Pro Asp Ser
               100                 105                 110
   Leu Leu Arg Ile Ser Glu Leu Met Glu Glu Glu Leu Pro Glu Glu Tyr
           115                 120                 125
   Arg Lys Asp Ile Val Val Leu Ser Gly Pro Ser His Ala Glu Glu Val
       130                 135                 140
   Gly Leu Arg His Pro Thr Thr Val Thr Ser Ser Ser Lys Asn Ile Lys
   145 150 155 160
   Ala Ala Glu Ala Val Gln Asp Leu Phe Met Asn Gln His Phe Arg Val
   165 170 175
   Tyr Thr Asn Pro Asp Met Ile Gly Val Glu Ile Gly Gly Ala Leu Lys
   180 185 190
   Asn Ile Ile Ala Leu Ala Ala Gly Ile Thr Asp Gly Leu Gly Tyr Gly
   195 200 205
   Asp Asn Ala Lys Ala Ala Leu Ile Thr Arg Gly Leu Ala Glu Ile Ala
   210 215 220
   Arg Leu Gly Thr Lys Met Gly Gly Asn Pro Leu Thr Phe Ser Gly Leu
   225 230 235 240
   Thr Gly Val Gly Asp Leu Ile Val Thr Cys Thr Ser Val His Ser Arg
   245 250 255
   Asn Trp Arg Ala Gly Asn Leu Leu Gly Lys Gly Tyr Lys Leu Glu Ala
   260 265 270
   Val Leu Asp Lys Met Gly Met Val Val Glu Gly Val Arg Thr Thr Lys
   275 280 285
   Ala Ala Tyr Gln Leu Ser Gln Lys Tyr Gln Val Lys Met Pro Ile Thr
   290 295 300
   Glu Ala Leu His Gln Val Leu Phe Asn Gly Gln Lys Val Glu Thr Ala
   305 310 315 320
   Val Glu Ser Leu Met Ala Arg Val Lys Thr His Glu Met Glu Asp Leu
   325 330 335
   Val Asn Thr Phe Glu Asn Arg Val Lys
   340 345
<210> 3
   <211> 25
   <212> DNA
   <213> Artificial Sequence
<220>
   <223> Primer
<400> 3
   gctgttaagc cgccgagctt cgttg 25
<210> 4
   <211> 28
   <212> DNA
   <213> Artificial Sequence
<220>
   <223> Primer
<400> 4
   taatcccata gcaccaagcg caaaccac 28
<210> 5
   <211> 23
   <212> DNA
   <213> Artificial Sequence
<220>
   <223> Primer
<400> 5
   cgacaaaagc cattcgggaa gtg 23
<210> 6
   <211> 21
   <212> DNA
   <213> Artificial Sequence
<220>
   <223> Primer
<400> 6
   tccgcgtcta tacaaatccc g 21
<210> 7
   <211> 21
   <212> DNA
   <213> Artificial Sequence
<220>
   <223> Primer
<400> 7
   cgtaggcgat ttaatcgtga c 21
<210> 8
   <211> 24
   <212> DNA
   <213> Artificial Sequence
<220>
   <223> Primer
<400> 8
   aatgaaagcg tctagattga aagg 24
<210> 9
   <211> 28
   <212> DNA
   <213> Artificial Sequence
<220>
   <223> Primer
<400> 9
   catgtttgat tggtaccttt ttattttc 28
<210> 10
   <211> 21
   <212> DNA
   <213> Artificial Sequence
<220>
   <223> Primer
<400> 10
   aagcgaaggt acccctcttt g 21
<210> 11
   <211> 32
   <212> DNA
   <213> Artificial Sequence
<220>
   <223> Primer
<400> 11
   cctatttgaa tatgacatct ctagaaaatt tc 32
<210> 12
   <211> 25
   <212> DNA
   <213> Artificial Sequence
<220>
   <223> Primer
<400> 12
   cgaatccggc acgcttgtgg atttg 25
<210> 13
   <211> 29
   <212> DNA
   <213> Artificial Sequence
<220>
   <223> Primer
<400> 13
   ccgtcccatc attgcatgcg ttatatttc 29
<210> 14
   <211> 34
   <212> DNA
   <213> Artificial Sequence
<220>
   <223> Primer
<400> 14 34
   gctaaaatct attattccgc ggttcagcaa tcgg 34
<210> 15
   <211> 30
   <212> DNA
   <213> Artificial Sequence
<220>
   <223> Primer
<400> 15
   gtccattcac tatccgcggt cccttttcag 30
<210> 16
   <211> 24
   <212> DNA
   <213> Artificial Sequence
<220>
   <223> Primer
<400> 16
   gaaggtgtga cgtctgcgga tgaa 24

## Beschreibung

Die vorliegende Erfindung betrifft ein mikrobielles Expressionssystem zur Produktion von Polypeptiden basierend auf der Nutzung von extrachromosomaler DNA, wobei zur Selektion der Wirtszellen keine Antibiotikamarkergene (Gene, deren abgeleiteten Proteine der Zelle Resistenz gegen ein Antibiotikum verleihen, auch Antibiotikaresistenzgene, Resistenzgene, Antibiotikamarker oder Antibiotika-Selektionsmarker genannt), sondern DNA-Sequenzen, die Glycerin-3-phosphatdehydrogenase (auch NAD(P)H-abhängige Dihydroxyacetonphosphatreduktase, NAD(P)H-abhängige Glycerin-3-Phosphatdehydrogenase, Glycerin-3-Phosphatdehydrogenase (NADP), Glycerin-3-phosphatsynthase, biosynthetische Glycerin-3-phosphatdehydrogenase, L-Glycerin-3-Phosphat:NAD(P) oxidoreduktase genannt) codieren, verwendet werden und so die Produktion des gewünschten Polypeptids, z.B. der Xylanase, vollständig ohne Zugabe von Antibiotika auskommt. Das Expressionssystem ist frei von Antibiotikaresistenzgenen. Die Erfindung betrifft ferner eine DNA-Sequenz, die ein Polypeptid mit Glycerin-3-phosphatdehydrogenaseaktivität codiert sowie ein Polypeptid mit Glycerin-3-phosphatdehydrogenaseaktivität.

In großer Menge benötigte Polypeptide und Enzyme werden heutzutage meist durch Fermentation von Mikroorganismen gewonnen. Hierbei kommen zwei Gruppen von Mikroorganismen zur Anwendung - i) solche Mikroorganismen, die die interessierenden Proteine natürlicherweise bilden, und ii) gentechnisch modifizierte Mikroorganismen. Die zur Modifikation der Mikroorganismen notwendigen gentechnischen Verfahren sind im Stand der Technik seit langem bekannt. Das Prinzip hierfür besteht darin, dass Gene, die für die interessierenden Proteine codieren, in die Wirtszellen eingebracht werden und von den Wirtszellen transkribiert, translatiert, eventuell posttranslational modifiziert und gegebenenfalls durch die betreffenden Membranen in das Periplasma beziehungsweise das umgebende Medium sekretiert werden. Die interessierenden Polypeptide sind dann aus den betreffenden Zellen beziehungsweise den Kulturüberständen isolierbar.

Bei technischen Verfahren zur Herstellung von Polypeptiden werden zunächst die natürlichen Fähigkeiten der zur Produktion eingesetzten Mikroorganismen zur Synthese und gegebenenfalls zur Sekretion der Proteine ausgenutzt. Grundsätzlich werden als Systeme zur Herstellung von Polypeptiden solche ausgewählt, die kostengünstig in der Fermentation sind, die eine hohe Produktbildungsrate aufweisen und die eine korrekte Faltung, Modifizierung etc. des herzustellenden Polypeptids versprechen. Die hierzu etablierten Mikroorganismen sind entweder eukaryontischen Ursprungs wie z.B. filamentöse Pilze (*Aspergillen, Trichoderma, Penicillium)* und Hefen (z.B. *Saccharomyces, Hansenula, Pichia*), oder es werden Prokaryonten verwendet wie z.B. *E. coli, Bacillen, Lactobacillen, Staphylococcen, Streptomyceten oder Pseudomonaden.*

Die Wirtschaftlichkeit eines biotechnologischen Verfahrens hängt entscheidend von der erzielten Ausbeute des Polypeptids ab. Diese Ausbeute wird neben dem verwendeten Expressionssystem vom eingesetzten Herstellungsverfahren, insbesondere von den Fermentationsparametern und den Nährmedien bestimmt. Durch Optimierung des Expressionssystems und des Fermentationsprozesses können das Potential und die erreichbare Ausbeute deutlich gesteigert werden.

Genetisch modifizierte Mikroorganismen enthalten die neue genetische Information entweder in das Genom integriert, wie dies bei filamentösen Pilzen oder Hefen häufig der Fall ist, oder auf extrachromosomalen Elementen wie z.B. Plasmiden, die oft in Prokaryonten oder auch Hefen zur Anwendung kommen. Erstere Konstrukte, bei denen die neue genetische Information in das Wirtsgenom integriert wird, sind auch ohne Selektionsdruck sehr stabil. Der Nachteil dieser Methode bei Prokaryonten ist, dass nach der Transformation nur eine Kopie des Gens im Wirt vorhanden ist und sich die Integration weiterer Kopien desselben Gens zur Erhöhung der Produktbildungsrate über den Gendosiseffekt methodisch sehr aufwändig gestaltet. Eine Lösung zu diesem Ansatz findet sich in EP 0 284 126 B1, das das Problem der stabilen Mehrfachintegration eines Gens darüber löst, dass die Kopien des exogenen Gens, die in das Wirtszellgenom integriert werden sollen, durch für die Zelle lebenswichtige endogene, chromosomale DNA voneinander getrennt vorliegen. Aus der Patentanmeldung DD 277467 A1 geht ein Verfahren zur Herstellung von extrazellulären Enzymen hervor, das auf der stabilen, vorteilhafterweise mehrfachen Integration der für das interessierende Polypeptid codierenden Gene in das Bakterienchromosom beruht. Die Integration erfolgt hierbei durch Rekombination über homologe Bereiche. Zur Kontrolle erfolgreicher Integrationsereignisse dient ein auf dem Plasmid, mit dessen Hilfe die Gene in die Zelle eingeschleust werden, enthaltenes Erythromycin-Gen, das bei erfolgreicher Integration inaktiviert wird.

In der Anmeldung WO 96/23073 A1 wird ein auf Transposition basierendes System zur Integration von mehreren Kopien eines interessierenden Gens in das Bakterienchromosom offenbart, das dadurch gekennzeichnet ist, dass die Markergene des Vektors während oder nach der Integration deletiert werden, und die erhaltenen Stämme somit frei von einem Markergen sind. Nach dieser Schrift wird ein Marker nur während der Konstruktion des betreffenden Bakterienstammes benötigt.

Ein System zur Erhöhung der Kopienzahl von bestimmten, in ein bakterielles Chromosom integrierten Genen wird in der Anmeldung WO 01/90393 A1 offenbart.

Bei der Nutzung von extrachromosomaler DNA zur Erzeugung eines genetisch modifizierten Mikroorganismus wird das interessierende Gen auf ein autonom replizierendes Element, zum Beispiel ein Plasmid, übertragen und episomal im Wirtsorganismus gehalten. Vorteilhaft für die Ausbeute des vom interessierenden Gen codierten Polypeptids wirkt sich dabei der Gendosis-Effekt über die üblicherweise hohe Zahl von Plasmidkopien pro Zelle aus. Nachteilig ist die Tatsache, dass über die gesamte Kulturzeit hinweg ein Selektionsdruck ausgeübt werden muss, um die extrachromosomalen Elemente stabil in den Zellen zu halten. Standardmäßig erfolgt dies über den Zusatz von Antibiotika in das Kulturmedium. Da sich das Gen, mit dem der Mikroorganismus Resistenz gegen das Antibiotikum erhält, auf dem extrachromosomalen Element befindet, können nur die Zellen, die ein solches Element aufweisen, wachsen. Das interessierende Gen wird in hoher Kopienzahl in den Zellen gehalten, indem es auf dem gleichen Plasmid, mit dem die Wirte Resistenz gegen das Antibiotikum/die Antibiotika erhalten, lokalisiert ist. Durch den Einsatz von Antibiotika als Selektionsdruck kann der Verlust des Plasmids aufgrund segregativer oder struktureller Instabilität verhindert werden (Bron und Luxen, 1985, Plasmid 14, 235-244). Auf diese Weise können auch größere Plasmide stabil in der Zelle gehalten werden, und die Zelle behält die Fähigkeit, das gewünschte Polypeptid zu produzieren. Generell findet ein Verlust von extrachromosomaler DNA sehr leicht statt, besonders wenn es sich um für diesen Organismus fremde DNA handelt. Auch bei Bakterien, die natürlicherweise Plasmide enthalten, ist die Anwendung von Selektionsdruck oft sinnvoll, da die natürlich vorkommenden, extrachromosomalen Elemente häufig nur in niedrigen Kopienzahlen vorliegen, für eine wirtschaftlich hohe Produktionsrate aber eine hohe Kopienzahl notwendig ist. Diese hohe Kopienzahl kann jedoch üblicherweise nur über einen Selektionsdruck aufrechterhalten werden.

Der Einsatz von Antibiotikaresistenzen als Selektionsmarker wird in den letzten Jahren zunehmend kritisch betrachtet. Zum einen ist der Einsatz von Antibiotika recht teuer, insbesondere wenn die Resistenz auf einem das Antibiotikum abbauenden Enzym beruht, so dass das Antibiotikum während der gesamten Kultivierung zugeführt werden muss. Zum anderen trägt ihr weitverbreiteter Einsatz, der sich auch auf andere Technik-Gebiete und auf die Medizin erstreckt, zur Ausbreitung der Resistenzgene auf andere, auch pathogene Stämme bei, was negative Folgen in der Krankheitsbekämpfung haben könnte.

Im Stand der Technik sind inzwischen auch antibiotikafreie Selektionssysteme entwickelt worden. So werden beispielsweise in der Publikation von Herrero et al. (1990, J. Bacteriol. 172, 6557-6567) Resistenzen gegen Herbizide und Schwermetalle als Selektionsmarker beschrieben. Gegen den Einsatz dieser Verbindungen sprechen jedoch dieselben Bedenken wie gegen Antibiotika.

Eine andere angewendete Methode, um Plasmide stabil in der Zelle zu halten, besteht in der episomalen Komplementierung auxotropher Stämme. In diesem Fall werden Gene vom Genom des Produktionsstammes entfernt oder inaktiviert, die für essentielle Stoffwechselfunktionen codieren. Die dadurch auxotrophen Wirtsstämme können nur dann wachsen, wenn die Stoffwechselfunktion alternativ wiederhergestellt wird. So können z.B. das notwendige Stoffwechselprodukt dem Medium zugegeben werden, wenn der Stamm in der Lage ist, diesen Metaboliten aufzunehmen, oder das Gen, das auf dem Wirtsgenom ausgeschaltet ist und das die essentielle Funktion codiert, episomal zur Verfügung gestellt werden. Vorteilhafterweise geschieht dies auf dem Plasmid, das auch das interessierende Gen für eine Polypeptidproduktion trägt. Das Patent EP 0 284 126 B1 nennt für Auxotrophie-Selektionsmarker die Stoffwechselgene *leu, his, trp* oder ähnliche, insbesondere solche aus Aminosäuresynthesewegen.

In der Praxis ist der Einsatz solcher Auxotrophien als Selektionsmarker aber bisher sehr schwierig, da insbesondere in industriellen Fermentationsmedien fast alle notwendigen Substanzen wie Aminosäuren und Vitamine in ausreichenden Mengen verfügbar sind, und die betreffenden Zellen die Unfähigkeit zur Synthese eines bestimmten Metaboliten über die Aufnahme dieses Metaboliten aus dem Nährmedium ausgleichen können.

Die industriell genutzten Fermentationsmedien enthalten in der Regel Bestandteile, die Abfallstoffe anderer, oft fermentativer Prozesse sind, z.B. Rückstände von Getreide aus der Ethanolherstellung (distillers spent grain), Rückstände von Mais aus der Stärkegewinnung (Maisquellpulver oder Maisquellwasser) oder Rückstände von Kartoffeln aus der Stärkegewinnung (Kartoffelschlempe). Diese Bestandteile dienen sowohl als Kohlenstoff- (C-) als auch als Stickstoff- (N-) Quellen und sind oft reich an z.B. Vitaminen oder Aminosäuren aufgrund der mikrobiellen Fermentation, die bei ihrer Gewinnung beteiligt war. Die industriell genutzten Fermentationsmedien sind folglich sehr komplex. Es ist daher schwierig, wenn nicht sogar unmöglich, auch bei Nutzung von auxotrophen Stämmen in diesen Medien einen Selektionsdruck aufrecht zu erhalten.

Ausnahmen sind bisher nur Auxotrophien für das essentielle Thymidin und das für *Bacillen* und gram-positive Mikroorganismen notwendige D-Alanin, die in industriellen Fermentationsmedien lediglich in Spuren oder gar nicht vorkommen und deshalb von den Mikroorganismen selber gebildet werden müssen. So bietet die Anmeldung EP 0 251 579 A1 die Lösung an, als Wirtsstämme solche einzusetzen, die hinsichtlich des für den Nucleotidstoffwechsel essentiellen Gens für die Thymidylat-Synthase defizient sind. Über einen Vektor kann demnach das Gen für eben diese Funktion (thyA aus *Escherichia coli* K12) zur Verfügung gestellt werden und den Gen-Defekt kurieren. Das Patent EP 0 185 512 B1 löst das Problem über die Insertion des dal-Gens (D,L-Alanin-Racemase) in das Plasmid unter Nutzung *dal*defizienter Wirtsstämme.

Eine weitere Lösung dieses Problems wurde in der Anmeldung WO 2004/078953 beschrieben. In ihr wurde erkannt, dass die an der Sekretion beteiligten essentiellen Faktoren für eine Selektion geeignet sind. Zur Selektion dient ein Gen, dessen abgeleitetes Protein als ein für die betreffende Zelle essentieller Faktor an der Protein-Translokation beteiligt ist, z.B. bei *Bacillus* die Proteine SecA, SecY, SecE, b-SRP, FtsY oder PrsA. Das bedeutet, dass der Ausfall eines solchen Faktors letal ist und damit eine Antibiotikum-ähnliche Selektion der rekombinanten Mikroorganismen erlaubt.

Insgesamt muss festgestellt werden, dass trotz jahrelanger Erfahrung zur Produktion von Polypeptiden durch biotechnologische Verfahren bisher kein praktikables System vorhanden ist, bei dem ohne Selektion über teure oder ökologisch bedenkliche Substanzen wie Antibiotika mit hoher Kopienzahl des interessierenden Gens produziert werden kann. Auch die verschiedenen Ansätze zur Selektion über Auxotrophiemarker haben wegen den in der Industrie verwendeten komplexen Nährmedien bisher zu dürftigen Ergebnissen geführt (WO 2004/078953) oder die Systeme enthalten auch weiterhin Antibiotikaresistenzgene.

Die Nutzung von definierten Medien aus gereinigten Bestandteilen (reine C- und N-Quellen als auch Vitamine, Aminosäuren und Mineralstoffe) ist für die Herstellung von industriell genutzten Polypeptiden, wie z.B. Enzymen in der Nahrungs-, Tierfutter- oder Waschmittelindustrie aus Kostengründen nicht machbar.

Der vorliegenden Erfindung liegt somit die Aufgabe zugrunde, ein Expressionssystem zur Produktion von Polypeptiden bereitzustellen, bei dem keine Selektion über teure und/oder umwelt- und/oder gesundheitsschädliche Stoffe erfolgt. Insbesondere soll eine Selektion nicht über Antibiotikaresistenzen erfolgen. Das erfindungsgemäße Expressionssystem soll einfach handhabbar sein und sich universell zur Expression beliebiger Zielpolypeptide eignen. Das erfindungsgemäße Expressionssystem soll sich auch zur Etablierung in beliebigen Wirtszellen eignen. Das erfindungsgemäße Expressionssystem soll ferner eine Selektion in industriell üblichen bzw. kostengünstigen Kulturmedien erlauben.

Gelöst wird diese Aufgabe durch ein Expressionssystem zur Produktion eines oder mehrerer Zielpolypeptids/Zielpolypeptide, umfassend eine Wirtszelle, in deren Genom die DNA-Sequenz, die Glycerin-3-phosphatdehydrogenase codiert, inaktiviert oder teilweise oder vollständig deletiert ist, und die mit einem extrachromosomalen Element, das eine DNA-Sequenz, die das bzw. die Zielpolypeptid(e) und Glycerin-3-phosphatdehydrogenase codiert, umfasst, transformiert worden ist, wobei sowohl das Wirtszellengenom als auch das extrachromosomale Element kein Antibiotikaresistenzgen tragen.

Überraschenderweise wurde gefunden, dass ein auf einem extrachromosomalen Element bereitgestelltes Glycerin-3-phosphatdehydrogenasegen in diesbezüglich auxotrophen Wirtszellen zur Selektion der entsprechenden Wirtszellen genutzt werden kann. Das extrachromosomale Element, das das Glycerin-3-phosphatdehydrogenasegen trägt, trägt ferner das Gen für das interessierende, zu produzierende Polypeptid.

Das Glycerin-3-phosphatdehydrogenasegen dient somit als Selektionsmarker zur Stabilisierung des extrachromosomalen Elements in den auxotrophen Wirtszellen. Diese Stabilisierung des extrachromosomalen Elements, das eine DNA-Sequenz, die das bzw. die Zielpolypeptid(e) und Glycerin-3-phosphatdehydrogenase codiert, umfasste, beruht auf der episomalen Komplementierung der auxotroph gemachten Wirtszelle. In dem Genom des Wirtsstammes wird eine DNA-Sequenz, die Glycerin-3-phosphatdehydrogenase codiert bzw. ein entsprechendes Gen inaktiviert. Dieses Gen codiert das Enzym Glycerin-3-phosphatdehydrogenase (auch NAD(P)H-abhängige Dihydroxyacetonphosphatreduktase; Glycerin-3-phosphatsynthase, biosynthetische Glycerin-3-phosphatdehydrogenase; Morbidoni et al., 1995, J. Bacteriol. 177 (20), 5899-5905; EC-Nummern 1.1.1.8, 1.1.1.94, hier auch u.a. NAD(P)H-abhängige Glycerin-3-Phosphatdehydrogenase, Glycerin-3-Phosphatdehydrogenase (NADP), L-Glycerin-3-Phosphat:NAD(P) oxidoreduktase). Die Glycerin-3-phosphatdehydrogenase katalysiert die Umsetzung von Dihydroxyacetonphosphat zu sn-Glycerin-3-phosphat unter Kopplung von NAD(P)H. sn-Glycerin-3-phosphat wiederum ist Ausgangsstoff für die Phospholipidsynthese der Zelle und damit zentraler Metabolit für die Zellmembransynthese. Je nach Organismus wird das Gen für die NAD(P)H-abhängige Glycerin-3-phosphatdehydrogenase unter anderem als gpsA (auch gol, gly, glyc genannt), gpd (gpd 1/2/3/A/A1/A2/h) oder dar1 bezeichnet. Generell handelt es sich um das Enzym, das unter physiologischen Bedingungen die Synthese von Glycerin-3-phosphat katalysiert und als Kofaktor üblicherweise NAD(P)H verwendet. Denkbar wäre auch ein entsprechendes Enzym, das einen anderen Kofaktor verwendet, z.B. FAD. Je nach Wirtsstamm werden das entsprechende Glycerin-3-phosphatdehydrogenase codierende Gen bzw. die Glycerin-3-phosphatdehydrogenasegene inaktiviert.

Durch die Deletion des Glycerin-3-phosphatdehydrogenasegens in dem Genom des Wirtsstammes wird dieser auxotroph für Glycerin-3-phosphat (G3P), kann aber wachsen, wenn das Nährmedium entsprechend supplementiert wird (mit G3P oder Glycerin) oder das entsprechende Gen episomal geliefert wird. Durch Insertion des Glycerin-3-phosphatdehydrogenasegens in das Plasmid, das auch die DNA-Sequenz für das interessierende Polypeptid trägt, wird das Plasmid stabil in der auxotrophen Wirtszelle gehalten. Gleichzeitig werden die auf Plasmiden üblicherweise vorkommenden Antibiotikaresistenzgene eliminiert. Ferner werden auch gegebenenfalls im Genom einer Wirtszelle vorhandene Antibiotikaresistenzgene eliminiert. Die Eliminierung der Antibiotikaresistenzgene erfolgt in an sich bekannter Weise, zum Beispiel vom Genom durch homologe Rekombination (s.u.) oder vom Plasmid durch Ausschneiden mit Hilfe geeigneter Restriktionsenzyme und anschließende Ligation.

Im Genom der Wirtszelle des erfindungsgemäßen Expressionssystems wird die jeweilige DNA-Sequenz, die Glycerin-3-phosphatdehydrogenase codiert, deletiert. Die Deletion kann teilweise oder vollständig sein. Jedenfalls muss die Deletion derart vorliegen, dass das Glycerin-3-phosphatdehydrogenasegen inaktiviert ist. Die Inaktivierung dieses Gens im Wirtsstamm erfolgt beispielsweise über homologe Rekombination mit einer inaktivierten oder (teilweise) deletierten Genkopie. Als Ergebnis dieses Rekombinationsereignisses wird die chromosomale Kopie des Gens funktionsunfähig. Bevorzugte Systeme sind darüber hinaus dadurch gekennzeichnet, dass die Inaktivierung des Glycerin-3-phosphatdehydrogenasegens auf dem Chromosom so erfolgt, dass eine spätere Rekombination zwischen der inaktivierten chromosomalen Genkopie und den homologen Bereichen auf dem kurierenden Komplementierungsvektor verhindert wird, vorzugsweise unter vollständigem Verlust eines in dem betreffenden chromosomalen Locus enthaltenen Gens oder Genabschnittes. Eine Rekombination und Integration des Komplementierungsvektors in das Genom des Wirtes, mit dem der Zelle die essentielle Funktion episomal zur Verfügung gestellt wird, würde die Inaktivierung wieder kurieren und somit den Selektionsdruck, über den das Plasmid stabil in der Zelle gehalten wird, aufheben. Dadurch könnte über die nachfolgenden Zellteilungen das eigentlich interessierende, zu exprimierende Gen verloren gehen oder nur noch in einer oder wenigen Kopien auf dem Chromosom vorliegen. Eine weitreichende oder vollständige Deletion während des Inaktivierungsschritts, die theoretisch auch stromaufwärts und stromabwärts liegenden DNA-Abschnitte mit einbinden kann, verhindert dies. Dabei ist zu beachten, dass die Bereiche stromaufwärts und stromabwärts vom *gpsA*-Gen Funktionen im Wirtsstamm aufweisen können, die für den Wirt ebenfalls essentiell sind.

Um nun eine homologe Rekombination zwischen dem durch das extrachromosomale Element bereitgestellten Glycerin-3-phosphatdehydrogenasegen und einem eventuell noch vorhandenen chromosomalen Gen auszuschließen, ist es notwendig, das Glycerin-3-phosphatdehydrogenasegen auf dem Wirtsgenom nicht nur durch einzelne Punktmutationen zu inaktivieren, sondern es weitgehend oder vollständig zu entfernen. Die Elimination des Gens vom Wirtsgenom erfolgt beispielsweise über homologe Rekombination mithilfe eines Deletionsvektors, der nur einen inaktiven Teil des Gens oder besser, nur die flankierenden Bereiche des Gens ohne das Gen selbst enthält, so dass die Genkopie auf dem Wirtsgenom gegen die auf dem Deletionsvektor vorhandene trunkierte Kopie ausgetauscht oder vollständig deletiert wird. Es ist wesentlich, dass dabei keine Antibiotikamarkergene zurückgelassen werden. Um das Gen vollständig vom Genom des Wirtes zu eliminieren, ist es notwendig, die das Gen stromaufwärts und stromabwärts flankierenden Bereiche aus dem Wirtsgenom zu isolieren und diese flankierenden Regionen ohne das Glycerin-3-phosphatdehydrogenasegen selbst in einen Deletionsvektor zu inserieren. Die homologe Rekombination findet dann statt zwischen den das Glycerin-3-phosphatdehydrogenasegen flankierenden Sequenzen, wobei das Glycerin-3-phosphatdehydrogenasegen vollständig vom Genom entfernt wird.

Erfindungsgemäß wird das auf dem Wirtschromosom deletierte bzw. inaktivierte Glycerin-3-phosphatdehydrogenasegen auf einem extrachromosomalen Element, das auch eine ein interessierendes Polypeptid codierende DNA-Sequenz trägt, der Zelle wieder zur Verfügung gestellt. Dabei kann sowohl dasselbe Gen, das in der jeweiligen Wirtszelle deletiert wurde, als auch ein entsprechendes Gen, das für NAD(P)H-abhängige Glycerin-3-phosphatdehydrogenase codiert, der Wirtszelle wieder zur Verfügung gestellt werden.

Die Erfindung betrifft somit auch einen Vektor zur Komplementierung des Genoms einer Wirtszelle, in dem die DNA-Sequenz, die Glycerin-3-phosphatdehydrogenase codiert, deletiert ist, umfassend eine DNA-Sequenz, die das bzw. die Zielpolypeptide codiert, sowie eine Expressionskassette, die eine DNA-Sequenz, die Glycerin-3-phosphatdehydrogenase codiert, umfasst, wobei der Vektor keine Antibiotikaresistenzgene enthält. Der Vektor kuriert somit die Inaktivierung der Glycerin-3-phosphatdehydrogenase codierenden DNA ("Komplementierungsvektor"), d.h. er liefert extrachromosomal eine aktive Glycerin-3-phosphatdehydrogenase-codierende Genkopie. Die Ausdrücke Vektor und Plasmid werden im Wesentlichen austauschbar verwendet. Als Vektoren können auch andere extrachromosomale Elemente wie z.B. Phagen, Phagmide oder Transposons dienen.

Als Vektor wird ein Plasmid bevorzugt, das eine hohe Kopienzahl in der Wirtszelle aufrecht erhält. Besonders vorteilhaft ist es, wenn es sich bei dem Plasmid um eines sich in mehrfacher (beispielsweise 10 bis 30 Plasmide pro Zelle), vorzugsweise in vielfacher Kopienzahl (mehr als 30 Plasmide pro Zelle) etablierendes Plasmid handelt. Je mehr Plasmidkopien vorliegen, desto höher ist aufgrund des Gendosiseffekts die Ausbeute an dem gewünschten Proteinprodukt.

Der Komplementierungsvektor soll erfindungsgemäß keine Antibiotikaresistenzgene enthalten. Des Weiteren enthält der Komplementierungsvektor neben den interessierenden Sequenzen für eine Polypeptidproduktion eine Expressionskassette mit dem Glycerin-3-phosphatdehydrogenasegen.

Hierbei wird vorzugsweise die endogen in den Wirtszellen vorhandene, deletierte Glycerin-3-phosphatdehydrogenase-codierende Sequenz verwendet. Es können aber auch ein Enzym gleicher Funktion codierende Sequenzen aus anderen Organismen, vorzugsweise aus verwandten Stämmen eingesetzt werden, sofern sie in der Lage sind, den betreffenden Defekt zu kurieren und dabei ein System bereitzustellen, das ohne weiteren Selektionsdruck eine hohe Produktivität des interessierenden Proteins gewährleistet. Ein Verlust dieser extrachromosomalen DNA wäre bei Wachstum in Minimalmedium für die auxotrophe Wirtszelle letal, so dass eine solche Zelle gezwungen ist, bei jeder Zellteilung dieses extrachromosomale Element an die Folgegeneration weiterzugeben. Bei dem erfindungsgemäßen System herrscht ein endogener Selektionsdruck, solange der rekombinante Stamm in einem Medium ohne Bereitstellung von Glycerin-3-phosphat oder Glycerin wächst. Die Gabe eines Antibiotikums ist nicht notwendig, um den Verlust des Vektors mit dem zu exprimierenden Gen zu verhindern.

Die Expressionskassetten, die zur erfindungsgemäßen Einschleusung der eine Aktivität einer Glycerin-3-phosphatdehydrogenase-codierenden DNA-Sequenz bzw. eines offenen Leserasters in eine Wirtszelle verwendet werden können, umfassen bevorzugt eine Transkriptionsstartregion, die mit dem offenen Leseraster verknüpft ist. Eine derartige Expressionskassette kann eine Vielzahl von Restriktionsschnittstellen zur Insertion des offenen Leserasters und/oder anderer DNA, z.B. einer Transkriptions-Regulationsregion enthalten. Die Transkriptionskassette umfasst in der 5'→3'-Richtung der Transkription eine Transkriptions- und Translationsstartregion, die DNA-Sequenz, die für die Glycerin-3-phosphatdehydrogenase-Aktivität codiert, und eine Translations- und Transkriptionsstoppregion, die in einer mikrobiellen Zelle funktionell ist. Die Terminationsregion kann bezüglich der Transkriptioninitiationsregion nativ sein, kann bezüglich der DNA-Sequenz von Interesse nativ sein oder kann von einer beliebigen anderen Quelle abgeleitet sein.

Der Ausdruck "offenes Leseraster" (ORF) bezeichnet die Aminosäuresequenz, die zwischen den Translationsstart- und -stop-Codons einer codierenden Sequenz codiert wird. Die Ausdrücke "Start-Codon" und "Stop-Codon" bezeichnen eine Einheit aus drei benachbarten Nucleotiden (Codons) in einer codierenden Sequenz, die den Kettenstart und -stop der Proteinsynthese (mRNA-Translation) spezifizieren.

"Funktionelle Verknüpfung" bezeichnet im Zusammenhang mit einer Nucleinsäure eine Verbindung als Teil des gleichen Nucleinsäuremoleküls in geeigneter Positionierung und Orientierung zum Transkriptionsstart des Promotors. DNA in funktioneller Verknüpfung an einen Promotor befindet sich unter der Transkriptions-Initiations-Regulation des Promotors. Codierende Sequenzen können funktionell mit der Regulatorsequenz in Sense- oder Antisense-Orientierung verknüpft sein. Bei Bezugnahme auf Polypeptide bedeutet funktionelle Verknüpfung die Verbindung als Teil desselben Polypeptids, d.h. über Peptidylbindungen.

Erfindungsgemäß können beliebige Promotoren verwendet werden, solange sie das erfindungsgemäße System stabil halten. In der bevorzugten Ausführungsform werden schwache und konstitutive Promotoren genommen. Promotor bezeichnet die Nucleotidsequenz üblicherweise stromaufwärts (5') bezüglich der codierenden Sequenz und kontrolliert die Expression der codierenden Sequenz, indem die Erkennung für die RNA-Polymerase und anderer Faktoren, die für die richtige Transkription erforderlich sind, bereitgestellt wird. Der erfindungsgemäß verwendete Promotor kann einen Minimalpromotor umfassen, d.h. eine kurze DNA-Sequenz aus einer TATA-Box und anderen Sequenzen, die die Transkriptionsstartstelle spezifizieren, an die Regulatorelemente zur Kontrolle der Expression angefügt sind.

Mit dem erfindungsgemäßen Expressionssystem kann das interessierende Polypeptid mit höherer, mindestens jedoch gleicher Ausbeute produziert werden, ohne dass Antibiotika zu irgendeinem Zeitpunkt der Produktion zugegeben werden müssen, und ohne dass Antibiotikamarkergene vorhanden sind, und ohne dass Antibiotika oder Antibiotikaresistenzgene im Proteinprodukt vorkommen. Durch den Einsatz des erfindungsgemäßen Expressionssystems können die Produkte daher auch in Applikationen eingesetzt werden, in denen die Anwesenheit von ganzen oder Anteilen von Antibiotikamarkergenen nicht zulässig oder nicht erwünscht ist.

Die Erfindung betrifft ferner die Verwendung des Expressionssystems zur antibiotikafreien Produktion des Zielpolypeptids. Dabei wird das Expressionssystem in einem Medium, das frei von Glycerin-3-phosphat bzw. Glycerin-3-phosphat-liefernden Verbindungen ist, gezüchtet. Beispiele für solche Medien sind vorstehend ausgeführt. Die Züchtung des Expressionssystems erfolgt in an sich bekannter Weise.

Das erfindungsgemäße Expressionssystem eignet sich für beliebige Wirtszellen. Generell kann das erfindungsgemäße Expressionssystem für praktisch alle industriell zur fermentativen Proteinherstellung bedeutenden Wirtszellen verwendet werden. Beispiele sind gram-positive Wirtsorganismen, zum Beispiel der Gattung *Staphylococcus, Corynebacterium* oder *Bacillus,* insbesondere die Spezies *Staphylococcus camosus, Corynebacterium glutamicum, Bacillus subtilis, B. licheniformis, B. amylotiquefaciens, B. brevis, B. globigii, B. megaterium, B. clausii* oder *B. lentus,* und ganz besonders Derivate der Stämme *B. licheniformis* oder *B*. *amyloliquefaciens.*

Bevorzugt ist die Verwendung von Bacillus-Stämmen als Wirtszellen. Besonders bevorzugt ist die Verwendung von *Bacillus amyloliquefaciens.* In *Bacillus amyloliquefaciens* liegt das Glycerin-3-phosphatdehydrogenasegen als *gpsA* vor. Überraschenderweise wurde gefunden, dass sich das erfindungsgemäße Expressionssystem in *Bacillus amyloliquefaciens* etablieren lässt, ohne dass sich die Kopienzahl des Produktionsplasmids im Vergleich zu dem analogen Antibiotikamarkergen-haltigen Plasmid (pUB110-Derivat) verringert. Vielmehr konnte mit einem derart transformierten *B*. *amyloliquefaciens* Wirtsstamm (*GpsA*⁻) sogar im Vergleich zu dem mit dem Ausgangsplasmid mit Antibiotikamarkergen (pIK91 abgeleitet aus pUB110, siehe EP 0 585 617 B1) transformierten Wirtsstamm (*GpsA*⁺) eine höhere Produktivität erreicht werden.

Es wurde ferner gefunden, dass das endogene *gpsA*-Gen von *Bacillus amyloliquefaciens* am besten durch Verwendung der dieses Gen flankierenden Sequenzen aus dem Genom von *Bacillus amyloliquefaciens* entfernt werden kann. Ferner ist es vorteilhaft, wenn das endogene *gpsA*-Gen als solches episomal zur Verfügung gestellt wird. Dazu wurde das bisher unbekannte *gpsA*-Gen aus *B. amyloliquefaciens* isoliert.,Obwohl Sequenz-Datenbanken wie z.B. EMBL (European Bioinformatics Institute (EBI), Cambridge, Großbritannien; http://www.ebi.ac.uk) oder GenBank (National Center for Biotechnology Information NCBI, National Institutes of Health, Bethesda, MD, USA; http://www.ncbi.nlm.nih.gov/) *gpsA*-Gendaten enthalten, zeigt ein Vergleich des erfindungsgemäßen Gens mit *gpsA*-Genen aus anderen Organismen nur eine 76%-Identität auf DNA-Ebene mit dem für *Bacillus subtilis* beschriebenen Gen bzw. 71% Identität mit dem Gen von *Bacillus licheniformis.*

Das Enzym Glycerin-3-phosphatdehydrogenase liefert in *Bacillen* sn-Glycerin-3-phosphat (G3P), einen essentiellen Metaboliten der Zellmembransynthese. Morbidoni et al. (1995) beschreiben, dass der G3P-Gehalt in der *Bacillus subtilis* Zelle überaus fein reguliert werden muss und dass das Gleichgewicht zwischen Synthese und Abbau von G3P in der Zelle überaus kritisch ist. Experimente von Freese et al. (1972. In: Halverson et al. (Hg.) Spores V, 212-221) zeigten, dass *B. subtilis* Zellen, in denen sich Glycerinphosphat aufgrund eines Defektes der katabolischen Glycerinphosphatdehydrogenase (Gof) ansammelt, schlechter wachsen als normale Zellen und dass die Sporulation unterdrückt wird.

Generell ist es wünschenswert, dass das interessierende Gen, das das zu produzierende Polypeptid codiert, in möglichst hoher Kopienzahl in der Wirtszelle vorliegt, um die Ausbeute über den Gendosiseffekt zu steigern. In einer bevorzugten Ausführung werden daher Plasmide verwendet, die mit zahlreichen Kopien in der Zelle vorliegen, wie z.B. Derivate von pUB110, das mit ca. 50 Kopien pro Zelle vorkommt (Gryczan et al., 1978, J. Bacteriol 134, 318-329). Die Einführung ebenso zahlreicher Kopien des komplementierenden Gens *gpsA* in die auxotrophe Wirtszelle könnte allerdings das empfindliche Gleichgewicht zwischen Synthese und Abbau von G3P stören.

Überraschenderweise wurde nun gefunden, dass diese für *Bacillus subtilis* beschriebenen Probleme bei *Bacillus amyloliquefaciens* unter den erfindungsgemäßen Bedingungen nicht auftreten. In einer bevorzugten Ausführung wird das *gpsA-*Gen dabei unter einen schwachen Promotor gestellt. Zyprian und Matzura (1986, DNA 5, 219-225) berichten, dass in dem Vektor pUB110 ein solcher Promotor natürlicherweise vorliegt. Stellt man das *gpsA*-Gen unter die Regulation dieses Promotors, wird trotz erhöhter Genkopienzahl im Vergleich zu einer nichtmodifizierten Bacillus-Zelle das delikate Gleichgewicht zwischen Synthese und Abbau von G3P in der Zelle offenbar aufrechterhalten. Da der Promotor außerdem konstitutiv ist, wird genügend Glycerin-3-phosphatdehydrogenase zu jedem Zeitpunkt im Zellzyklus von *Bacillus* zur Verfügung gestellt. Darüber hinaus kommt es mit diesem System sogar zu einer stabilen Aufrechterhaltung der hohen Kopienzahl in der *gpsA*-negativen Wirtszelle bei Wachstum in Komplexmedium. Dies erlaubt die Produktion von interessierenden Polypeptiden, die von dem komplementierenden Vektor ebenfalls codiert werden, in hohen Konzentrationen in den von der Industrie üblicherweise verwendeten Nährmedien.

Wie bereits ausgeführt, können erfindungsgemäß beliebige Promotoren verwendet werden, solange sie das erfindungsgemäße System stabil halten. Insbesondere sind schwache, konstitutive Promotoren bevorzugt, wie z.B. der o.g. von Zyprian und Matzura (1986) für das Plasmid pUB110 beschriebene Promotor. Auch der ptsH Promotor aus *B. subtilis* ist ein geeigneter schwacher und konstitutiver Promotor (Stülke und Hillen, 2000, Annu. Rev. Microbiol. 54, 849-80).

Wie vorstehend ausgeführt, wird in dem erfindungsgemäßen Expressionssystem das Glycerin-3-phosphatdehydrogenasegen auf dem Wirtschromosom inaktiviert. Die Unfähigkeit zur Synthese von Glycerin-3-phosphatdehydrogenase wird dadurch ausgeglichen, dass das entsprechende Gen episomal zur Verfügung gestellt wird. GpsA-negative-Bacillus *subtilis* Mutanten wurden durch klassische Mutation bereits früh hergestellt (Mindich, 1970, J. Mol. Biol. 49, 415-432). Bei diesen Mutanten handelt es sich um Allele des *gpsA*-Gens (Morbidoni et al., 1995), die wahrscheinlich nur Punktmutationen in dem *gpsA*-Gen oder dessen Regulation haben. Diese Stämme (und dieses Verfahren) sind für eine großtechnische Proteinproduktion nicht brauchbar, da es sowohl zur spontanen Rückmutation zur aktiven Form als auch zur Rekombination des intakten, episomal bereitgestellten *gpsA-*Gens mit dem defekten chromosomalen Gen auf Grund der langen Homologiebereiche kommen kann (vgl. auch Ostroff et al., 1984, Mol. Gen. Genet. 193, 299-305). Khasanov et al. (1992, Mol. Gen. Genet. 234, 494-497) zeigten, dass es bereits bei Homologien von 70 bp in *Bacillus* zur homologen Rekombination im Genom kommen kann. In einem solchen Falle könnten die Wirtszellen wieder prototroph werden und wären nicht mehr auf das Plasmid mit dem *gpsA*-Gen angewiesen. Dies wiederum könnte zur einfachen Integration oder zum Verlust des Plasmids führen, so dass auch das interessierende Polypeptid, dessen Gen ebenfalls auf dem Plasmid vorliegt, nicht mehr oder nur in geringen Mengen von der Wirtszelle gebildet werden kann.

Das *gpsA*-Gen sowie die Regionen stromaufwärts und stromabwärts des Gens sind für *Bacillus amyloliquefaciens* nicht beschrieben, es liegen aus den Datenbanken (z.B. EMBL, GenBank, SubtiList (Moszer et al., 1995, Microbiology 141, 261-268; Moszer, 1998, FEBS Lett. 430, 28-36; http://genolist.pasteur.fr/SubtiList/)) nur Daten zu *Bacillus subtilis* und zu weiter entfernt verwandten Mikroorganismen vor. Auch die Organisation des Genoms von *B*. *amyloliquefaciens* ist nicht bekannt. Gene, die auf dem Chromosom von *B*. *subtilis* in direkter Nachbarschaft des *gpsA*-Gens liegen, die also die flankierenden Bereiche darstellen, welche zur Erzeugung des auxotrophen Wirtes benötigt werden, können im Genom von *B. amyloliquefaciens* an völlig anderen Positionen vorkommen oder auch ganz fehlen. Es ist auch zu beachten, dass die Bereiche stromaufwärts und stromabwärts vom *gpsA*-Gen Funktionen im Wirtsstamm codieren können, die für den Wirt essentiell sind. Für *B. subtilis* sind stromaufwärts und stromabwärts Gene mit unbekannten Funktionen beschrieben, während für *B.* a*myloliquefaciens* keine Informationen über benachbarte Gene vorliegen. Es empfiehlt sich daher für *B. subtilis,* aber auch für *B. amyloliquefaciens* und andere Ba*cilli* die an das *gpsA*-Gen angrenzenden Bereiche vollständig zu erhalten, um keine weiteren, unbekannten und daher nicht komplementierbaren Funktionen im Genom zu zerstören. Eine vollständige, aber möglichst punktgenaue Deletion des *gpsA*-Gens, die keine Mutation oder Leserasterverschiebung in den möglicherweise angrenzenden Genen hervorruft, ist daher erfindungsgemäß anzustreben.

Eine homologe Rekombination zum präzisen Entfernen eines chromosomalen Gens oder Genfragments wird umso erfolgreicher sein, je länger die homologen Bereiche sind (vgl. Hamilton et al., 1989, J. Bacteriol. 171 (9), 4617-4622). Es wurde gefunden, dass die das *gpsA*-Gen flankierenden Bereiche aus *B. amyloliquefaciens* mit den entsprechenden Bereichen aus *B. subtilis -* wie in der Datenbank SubtiList (Moszer et al., 1995; 1998) beschrieben - nur eine Sequenzidentität von etwa 84% für den Bereich von ca. 1,3 kbp stromaufwärts, und eine Identität von etwa 69% für den Bereich von ca. 1,1 kbp stromabwärts aufweisen. Die Amplifizierung der flankierenden Bereiche auf der chromosomalen DNA des Wirtsstammes *B. amyloliquefaciens* war daher äußerst schwierig, insbesondere, da möglichst lange homologe Bereiche für die Konstruktion des Deletionsvektors erzeugt werden sollten.

Die Deletion des Glycerin-3-phosphatdehydrogenasegens auf dem Wirtschromosom erfolgt beispielsweise mithilfe von Plasmidvektoren, die einen temperatursensitiven oder nicht in *Bacilli* funktionierenden Replikationsursprung besitzen und in die zusätzlich die in Frage kommenden (flankierenden) homologen DNA-Bereiche des zu deletierenden Gens eingefügt wurden (Deletionsvektor). Denkbar wäre allerdings auch eine reversible Inaktivierung, beispielsweise über Integration eines mobilen genetischen Elements, zum Beispiel eines Transposons, in das zu inaktivierende Gen.

In der Publikation von Vehmaanperä et al. (1991, J. Biotechnol. 19, 221-240) sind Verfahren zur Inaktivierung von Genen über einen Deletionsvektor beschrieben. Der Replikationsursprung dieses Deletionsvektors zeichnet sich durch seine Temperaturabhängigkeit aus. Dadurch ist es möglich, zunächst bei niedrigerer Temperatur auf eine erfolgreiche Transformation zu selektieren und anschließend durch Temperaturerhöhung einen Selektionsdruck auf eine erfolgreiche Integration auszuüben. Danach wird die Zelle vom Vektor, der jetzt die endogene Genkopie enthält, kuriert, so dass keine funktionierende Genkopie auf dem Chromosom mehr vorliegt. Es bleiben auch keine Vektorsequenzen, d.h. auch keine Antibiotikaresistenzgene in der Zelle zurück.

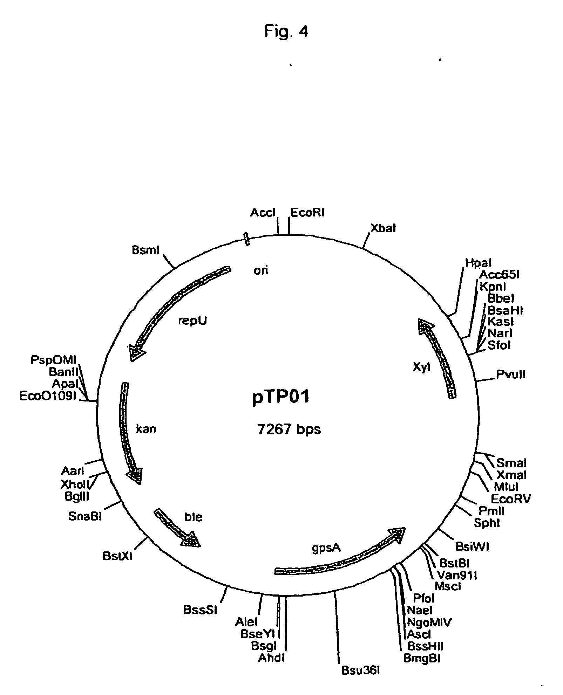

Die Erfindung betrifft somit ein Expressionssystem, welches eine DNA-Sequenz umfaßt, die für ein Polypeptid mit Glycerin-3-phosphatdehydrogenaseaktivität codiert, dadurch gekennzeichnet, dass die DNA-Sequenz ausgewählt ist aus a) DNA-Sequenzen, die eine Nucleotidsequenz gemäß SEQ ID NO:1 umfassen, b) DNA-Sequenzen, die eine Nucleotidsequenz, wiedergegeben durch die Nucleotide 1338 bis 2375 von SEQ ID NO:1, umfassen c) DNA-Sequenzen, die von dem Plasmid pTP01 mit der Plasmidkarte gemäß Figur 4 und hinterlegt unter der Hinterlegungsnummer DSM 18890 codiert werden, d) DNA-Sequenzen, die für die Proteinsequenz gemäß SEQ ID NO: 2 codieren, e) DNA-Sequenzen, die unter stringenten Bedingungen mit einer der DNA-Sequenzen gemäß a), b), c) oder d) hybridisieren, f) DNA-Sequenzen, die aufgrund der Degeneriertheit des genetischen Codes mit den DNA-/Nucleotidsequenzen gemäß a), b), c), d) oder e) verwandt sind, und g) komplementären Strängen zu den Sequenzen gemäß a) bis f). Die Erfindung betrifft ferner die Verwendung der vorstehenden DNA-Sequenz zur Herstellung des erfindungsgemäßen Expressionssystems. Überraschenderweise wurde gefunden, dass eine von *Bacillus amlyoliquefaciens* abgeleitete DNA-Sequenz, die für ein Polypeptid mit Glycerin-3-phosphatdehydrogenaseaktivität codiert, besonders vorteilhaft bei der Herstellung des erfindungsgemäßen Expressionssystems ist.

Der Grad der Sequenzidentität wird bezüglich der beanspruchten Sequenzen bevorzugt so bestimmt, dass man die Anzahl der Reste der kürzeren Sequenz, die an dem Vergleich beteiligt ist und die ein "entsprechendes" Gegenstück in der anderen Sequenz besitzt, ermittelt. Für die Zwecke der vorliegenden Erfindung wird die Identität dabei bevorzugt in üblicher Weise unter Verwendung der üblichen Algorithmen bestimmt. Erfindungsgemäß werden zum Vergleich nur die Nucleotide, die die reifen Proteine codieren bzw. die Aminosäuren der jeweiligen reifen Proteine herangezogen. Als homologe Sequenzen wurden erfindungsgemäß ähnliche, bevorzugt identische, Sequenzgegenstücke mit Hilfe von bekannten Computerprogrammen ermittelt. Ein Beispiel für ein derartiges Programm ist das Programm Clone Manager Suite, das den Programmteil Align Plus enthält und von Scientific & Educational Software, Durham, NC, USA vertrieben wird. Dabei wird ein Vergleich zweier oder mehrerer wie vorstehend definierter DNA- bzw. Aminosäuresequenzen durchgeführt unter der Option *local alignment* entweder nach der Methode FastScan - MaxScore oder nach der Methode Needleman-Wunsch unter Beibehaltung der Defaultwerte. Speziell wurde erfindungsgemäß zur Berechnung der Identität die Programmversion "Clone Manager 7 Align Plus 5" mit den Funktionen "Compare Two Sequences/Local/Fast Scan-Max Score/Compare sequences as DNA bases" bzw. für Aminosäuren "Compare Two Sequences/Local/ Fast Scan-Max Score/Compare sequences as Amino Acids" angewendet. Dabei wurden die aus den folgenden Quellen zugänglichen Algorithmen verwendet: Hirschberg (1975, Commun. Assoc. Comput. Mach. 18, 341-343); Myers und Miller (1988, CABIOS 4, 11-17); Chao et al. (1992, CABIOS 8, 481-487).

Die Erfindung betrifft ferner Additions- und/oder Deletionsmoleküle der vorstehenden Polypeptide mit Glycerin-3-phosphatdehydrogenase-Aktivität. So kann ein erfindungsgemäß modifiziertes Polypeptid mit Glycerin-3-phosphatdehydrogenase-Aktivität durch Anfügen weiterer Sequenzen am N-terminalen und/oder C-terminalen Ende oder im Molekül erhalten werden, wobei die so erhaltenen Polypeptide noch Glycerin-3-phosphatdehydrogenase-Aktivität aufweisen oder Glycerin-3-phosphatdehydrogenase-defiziente Stämme komplementieren können müssen. Dadurch können Hybridmoleküle hergestellt werden, die weitere vorteilhafte Eigenschaften besitzen.

Erfindungsgemäß können auch Sequenzabschnitte des Polypeptids mit Glycerin-3-phosphatdehydrogenase-Aktivität deletiert werden unter Beibehaltung der Glycerin-3-phosphatdehydrogenase-Aktivität. Die Mutationen, Elongationen und Verkürzungen können in an sich bekannter Weise nach auf dem Fachgebiet gut bekannten Verfahren durchgeführt werden.

Die Erzeugung solcher Varianten ist allgemein auf dem Fachgebiet bekannt. Beispielsweise können Aminosäuresequenz-Varianten der Polypeptide durch Mutation in der DNA hergestellt werden. Verfahren zur Mutagenese und Nucleotidsequenzveränderung sind im Stand der Technik gut bekannt (vgl. beispielsweise Kunkel, 1985, Proc. Natl. Acad. Sci. USA 82, 488-492, Kunkel et al., 1987, Methods Enzymol. 154, 367-382, US-Patent Nr. 4873192, Walker und Gaastra (Hg.), 1983, Techniques in Molecular Biology, Mac Millan Publishing Company, New York). Hinweise über geeignete Aminosäuresubstitutionen, die die biologische Aktivität des Proteins von Interesse nicht beeinträchtigen, finden sich in dem Modell von Dayhoff et al. (1978, Atlas of Protein Sequence and Structure. Natl. Biomed. Res. Found., Washington, D.C.). Konservative Substitutionen wie der Austausch einer Aminosäure gegen eine andere mit ähnlichen Eigenschaften sind bevorzugt. Diese Austausche lassen sich in 2 Hauptgruppen mit zusammen 4 Untergruppen einteilen und ein Austausch in jeder Untergruppe wird als konservativer Austausch bezeichnet, der bevorzugt nicht die Aktivität oder die Faltung des Proteins beeinflusst.

| | | |
|---|---|---|
| Aliphatisch | Nicht-Polar | G A P |
| | | I L V |
| | Polar und ungeladen | C S T M N Q |
| | Polar und geladen | D E |
| | | K R |
| Aromatisch | | H F W Y |

Die Ausdrücke "Protein", "Peptid" und Polypeptid" werden im Wesentlichen austauschbar verwendet. Ein Polypeptid oder Enzym mit Glycerin-3-phosphatdehydrogenase-Aktivität soll ein Enzym bezeichnen, das die NAD(P)H-gekoppelte Reduktion von Dihydroxyacetonphösphat zu Glycerin-3-phosphat katalysiert. Die Glycerin-3-phosphatdehydrogenase-Aktivität kann unter Verwendung an sich bekannter, beliebiger Testverfahren, bei denen eines dieser Substrate oder Produkte verwendet wird, bestimmt werden (Morbidoni et al., 1995; Bergmeyer, 1970, Methoden der enzymatischen Analyse. Verlag Chemie, 426-227).

Die hier beschriebene, erfindungsgemäße Art der Konstruktion von auxotrophen Wirtsstämmen ermöglicht es, einen einmal erzeugten auxotrophen Mikroorganismus-Stamm, dessen chromosomales Glycerin-3-phosphatdehydrogenasegen inaktiviert wurde, für immer neue Transformationen mit ähnlich aufgebauten, komplementierenden Vektoren zu verwenden, die jedes mal dieselbe den Gendefekt kurierende Funktion zur Verfügung stellen, aber jeweils verschiedene Gene für andere interessierende, zu produzierende Polypeptide tragen. Man verfügt somit über ein sehr praktisches und vielseitig einsetzbares Produktionssystem.

Der Sinn des erfindungsgemäßen Systems besteht darin, ein genetisches Element, das für die Produktion eines interessierenden Polypeptides gebraucht wird, ohne Antibiotikum-Selektionsdruck über mehrere oder viele Generationen hinweg stabil und in hoher Kopienzahl in der Zelle zu erhalten. Dieses Element ist eben das Plasmid, das sowohl das Glycerin-3-phosphatdehydrogenasegen trägt als auch das Gen für das zu produzierende Polypeptid.

Die Aufrechterhaltung dieses Selektionsdrucks ist für die Aufbewahrung der rekombinanten Produktionsstämme von Vorteil. Die inhärente Stabilität des Systems ist hingegen ausreichend, um im Produktionsprozess die hohe Kopienzahl aufrecht zu erhalten und damit eine hohe Produktivität zu gewährleisten.

Bedingt durch die hohe inhärente Stabilität bleibt das System auch ohne Anwendung von Selektionsdruck stabil, d.h. auch bei Anzucht in industriellem Medium (das möglicherweise Spuren von Glycerin enthält) in der Hauptkultur, nimmt die Ausbeute an Zielpolypeptid nicht über die Kultivierungsdauer ab, der Komplementierungsvektor wird also stabil in der Zelle gehalten.

Von ganz besonderem Interesse sind erfindungsgemäße Expressionssysteme, die auf bestimmte, durch die Kultivierung der Mikroorganismen hergestellte Produkte ausgerichtet sind, insbesondere Polypeptide oder Proteine wie z.B. hydrolytische Enzyme oder Oxidoreduktasen, besonders bevorzugt alpha-Amylasen, beta-Amylasen, maltogene Amylasen, CGTasen, Xylanasen, alpha-Galactosidasen, beta-Galactosidasen, Phospholipasen, Phosphatasen, Phytasen, Endoglucanasen, insbesondere endo-beta-1,4-Glucanasen, endo-beta-1,3(4)-Glucanasen, en-do-1,2-beta-Glucanasen und endo-1,3-alpha-Glucanasen, Cellulasen, Xylosidasen, Galactanasen, insbesondere Arabinogalactan-endo-1,4-beta-Galactosidasen und Arabinogalactan-endo-1,3-beta-Galactosidasen, Pektin-abbauende Enzyme, insbesondere Pektinasen, Pektinesterasen, Pektinlyasen, Polygalacturonasen, Arabananasen, Rhamnogalacturonasen, Rhamnogalacturonanacetylesterasen, Rhamnogalacturonan-alpha-Rhamnosidasen, Pektatlyasen und alpha-Galacturonidasen, Mannanasen, beta-Mannosidasen, Mannanacetylesterasen, Xylanacetylesterasen, andere Xylanasen, Arabinoxylanasen, proteolytische Enzyme wie Proteasen und Peptidasen, lipolytische Enzyme wie Lipasen, Digalactosid-Diglycerid-Esterasen und Cutinasen, und andere Enzyme wie Laccasen und Transglutaminasen.

Von besonderer Bedeutung ist daher die Nutzung eines erfindungsgemäßen Systems in großtechnischen Verfahren, vor allem zur Proteinherstellung. Verfahren zur Herstellung eines Proteins durch Kultivierung von Zellen eines Mikroorganismus-Stammes sind im Stand der Technik allgemein bekannt, wenn das Plasmid über einen Selektionsdruck, wie er mit Antibiotika aufgebaut werden kann, beruht.

Die Erfindung betrifft ferner ein Verfahren zur Produktion eines Polypeptids von Interesse unter Verwendung des erfindungsgemäßen Expressionssystems.

Von besonderer Bedeutung sind Proteinherstellungsverfahren, die dadurch gekennzeichnet sind, dass das interessierende Protein ins umgebende Medium sekretiert wird. Denn hierdurch wird die Aufarbeitung des erhaltenen Produkts wesentlich erleichtert. Eine erfindungsgemäß mögliche Alternative besteht jedoch auch darin, die betreffenden das Protein herstellenden Zellen im Anschluss an die eigentliche Produktion aufzuschließen und dadurch das Produkt zu erhalten.

Ein besonders vorteilhafter Aspekt besteht darin, dass eine Reihe verwandter Mikroorganismen dadurch erhalten wird, dass immer dieselbe Art der Inaktivierung und Kurierung durchgeführt, auf dem kurierenden Vektor aber jedesmal ein anderes Gen, das für ein anderes interessierendes Polypeptid codiert, bereitgestellt wird. Ein einmal erfolgreich entwickeltes System kann auf diese Weise auf unzählige weitere Produktionsprozesse übertragen werden.

### Figuren

Die beigefügten Figuren erläutern die Erfindung näher. Es zeigen
- Figur 1:: DNA-Sequenz des *gpsA*-Gens mit flankierenden Bereichen aus *Bacil- lus amyloliquefaciens;* Seq-ID Nr. 1 (kursiv: *gpsA*-Gen, ***kursiv-fett.*** putative RBS, **fett:** putativer Termina- tor)

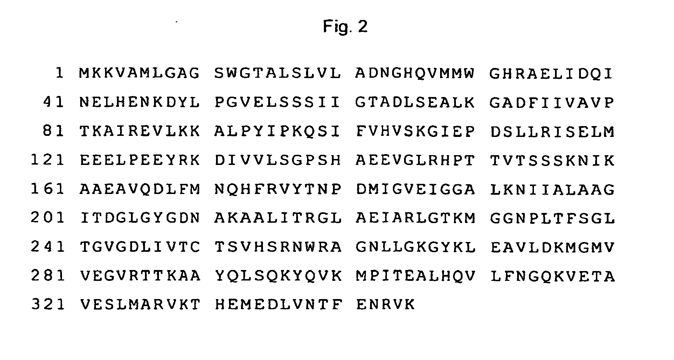

- Figur 2:: Aminosäure-Sequenz codiert durch das *gpsA*-Gen gemäß Figur 1; Seq-ID Nr. 2

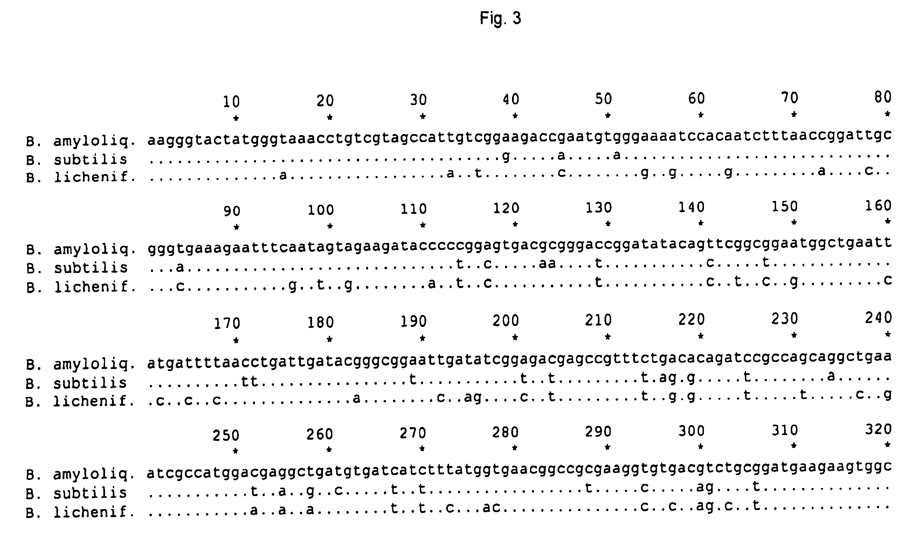

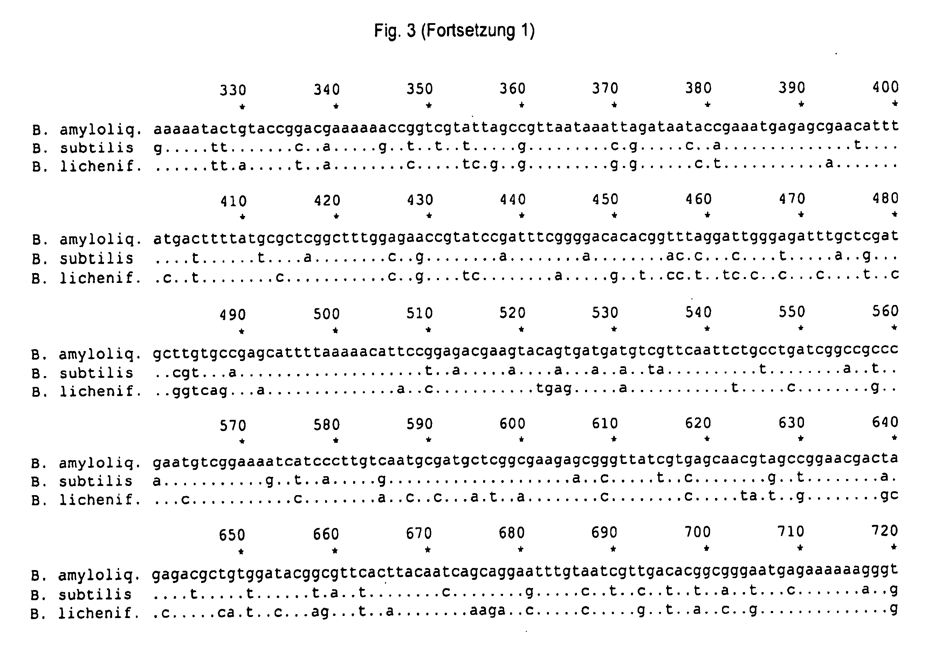

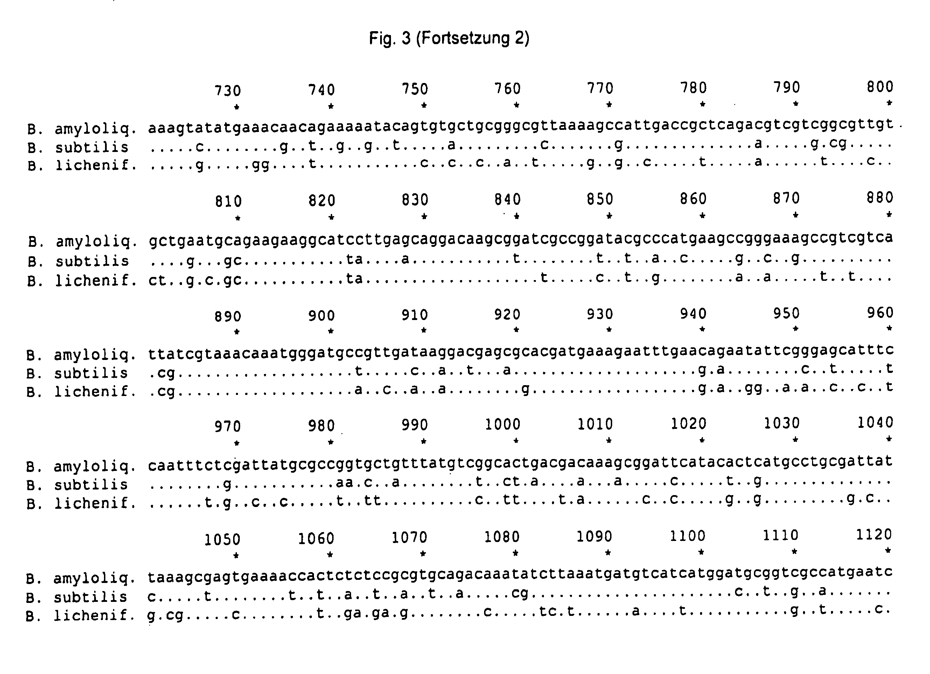

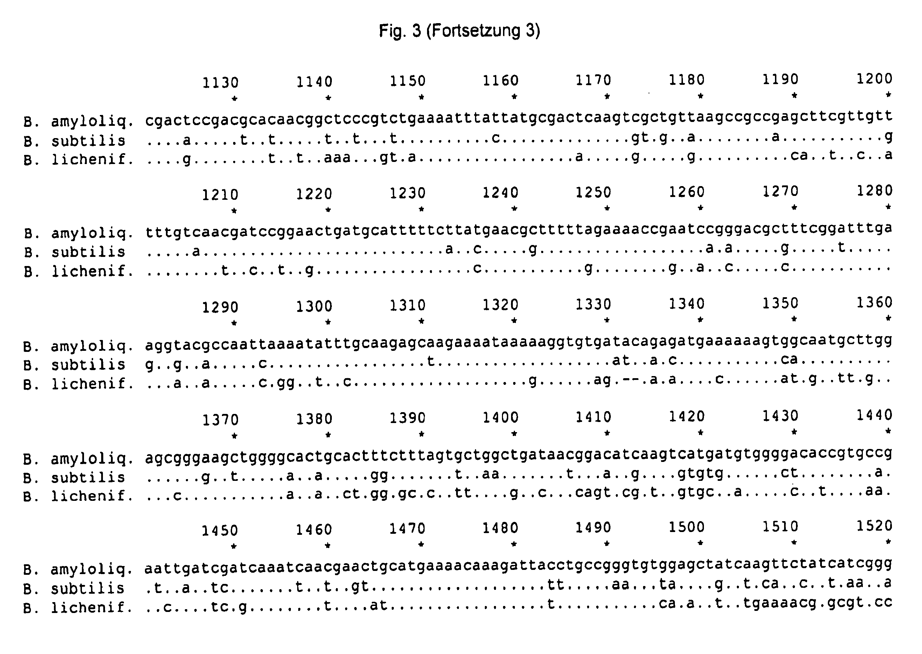

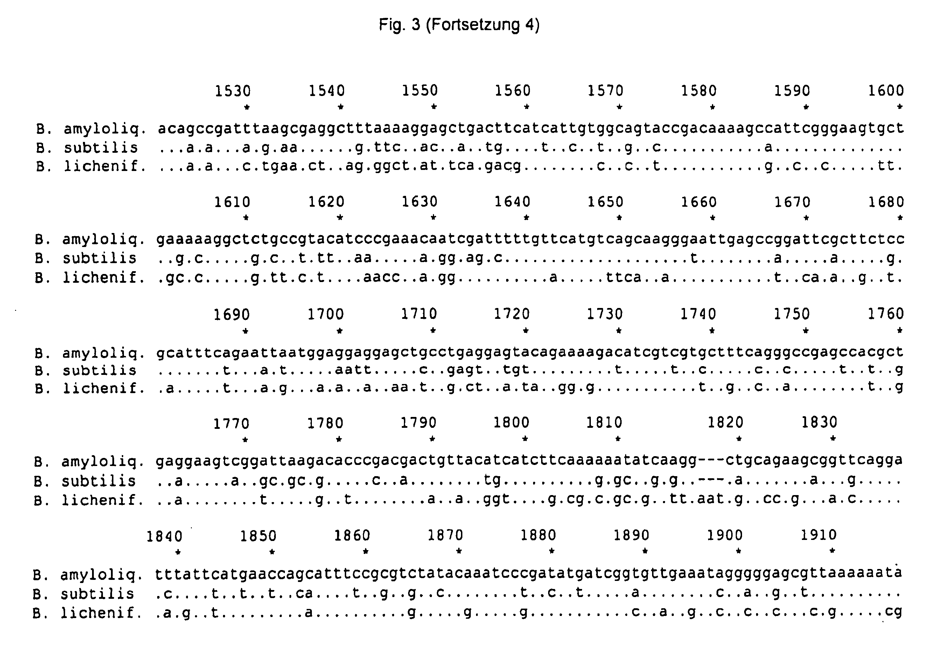

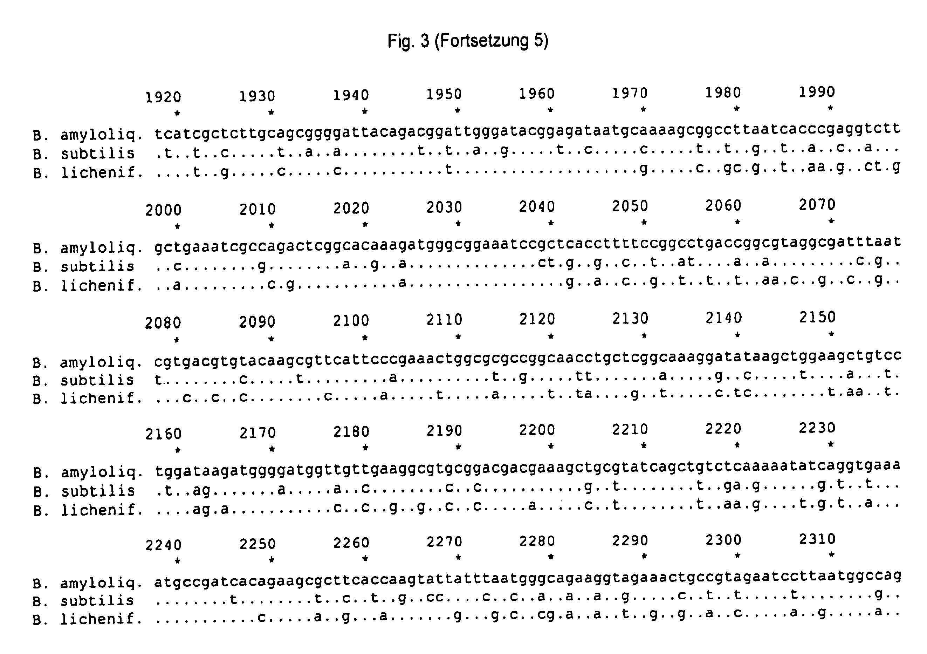

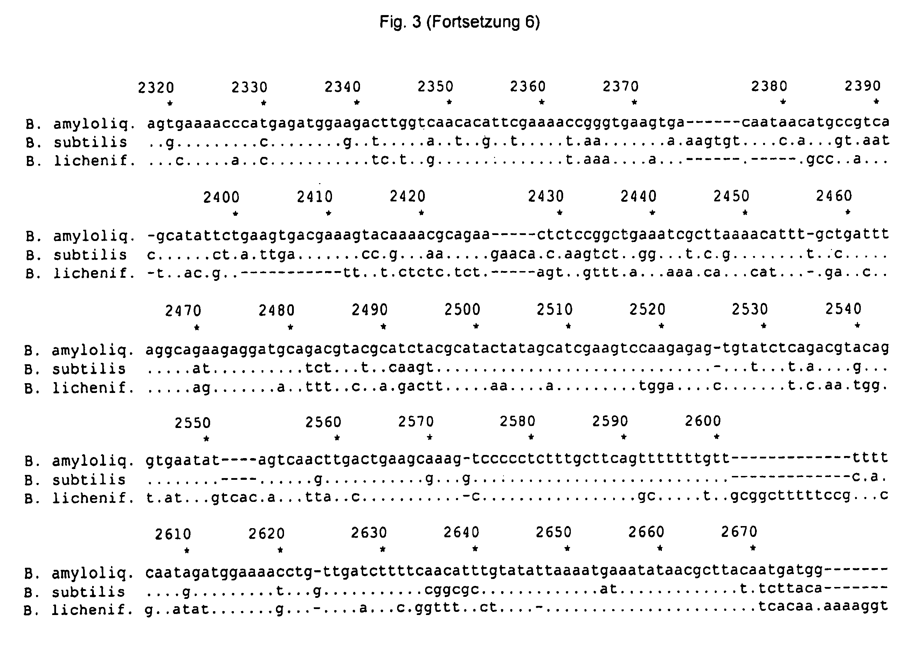

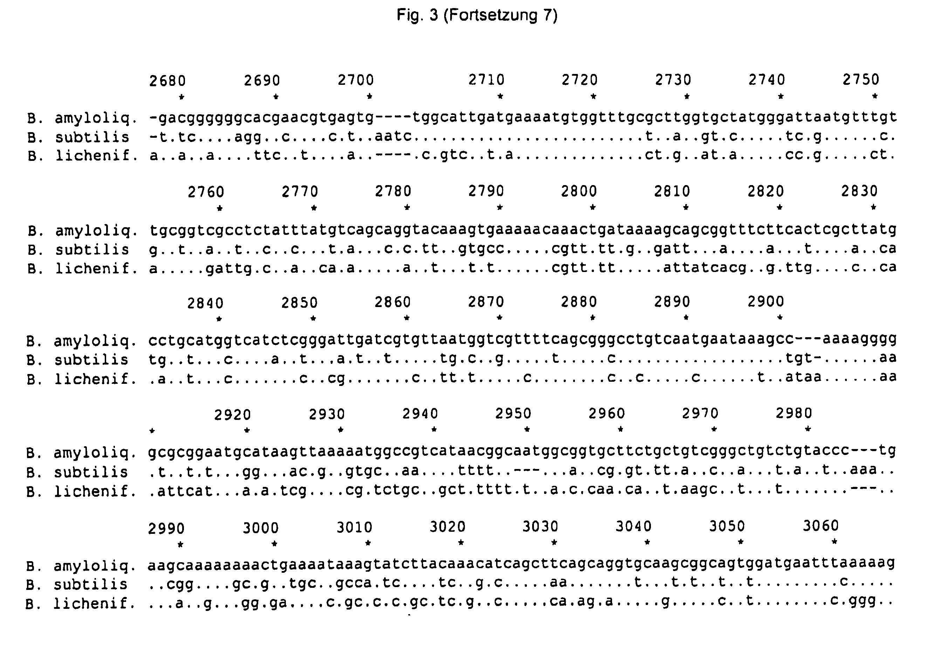

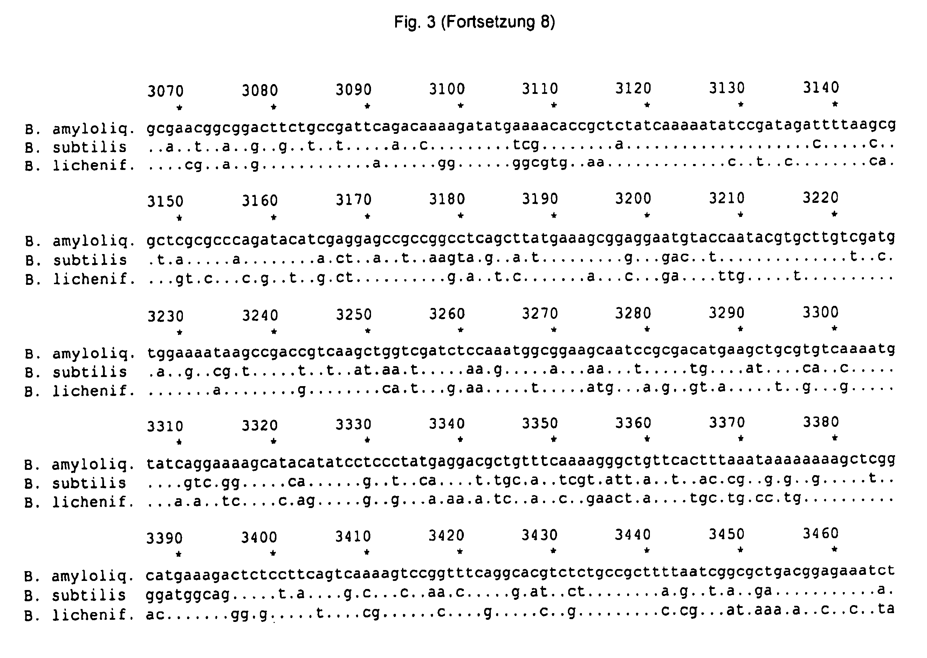

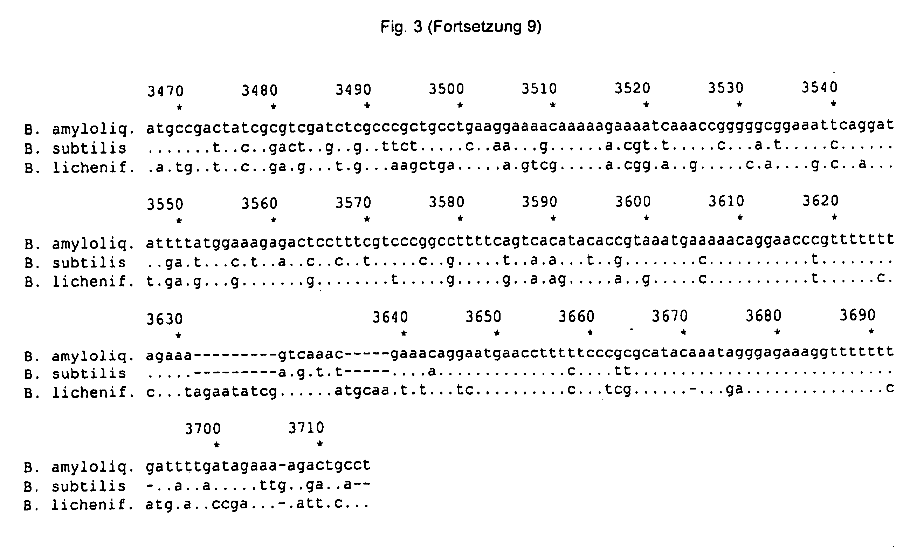

- Figur 3:: Alignment der DNA-Sequenz gemäß Figur 1 mit anderen bekannten *Bacillus gpsA*-Regionen *(B. amyloliq.: Bacillus amyloliquefaciens RH 1330; B. subtilis: Bacillus subtilis 168; B. lichenif.: Bacillus licheniformis A TCC 14580)*
- Figur 4:: Plasmidkarte von pTP01 mit dem hinterlegten *gpsA*-Gen

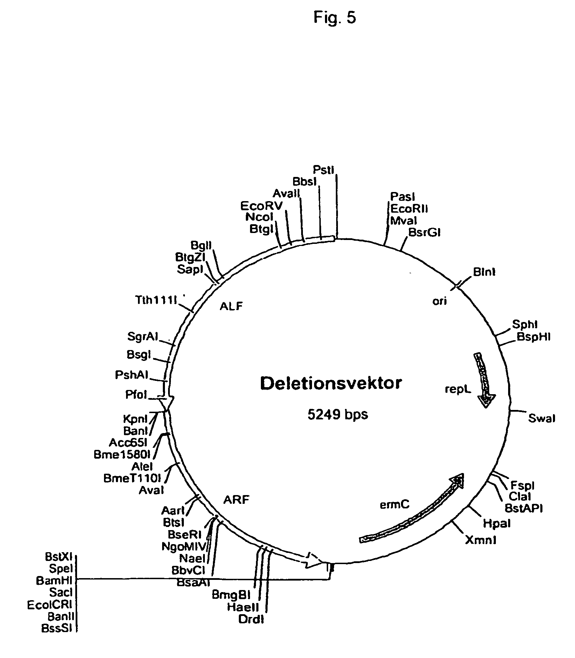

- Figur 5:: Plasmidkarte des Deletionsplasmids (ALF: Stromaufwärts des *gpsA*-Gens liegende Region auf dem Chro- mosom von *B. amyloliquefaciens* RH 1330; ARF: Stromabwärts des *gpsA*-Gens liegende Region auf dem Chromosom von *B*. *amylolique- faciens* RH 1330; ermC: das *ermC*-Gen codiert eine Adenin- Methylase, die Resistenz gegen Erythromycin verleiht).

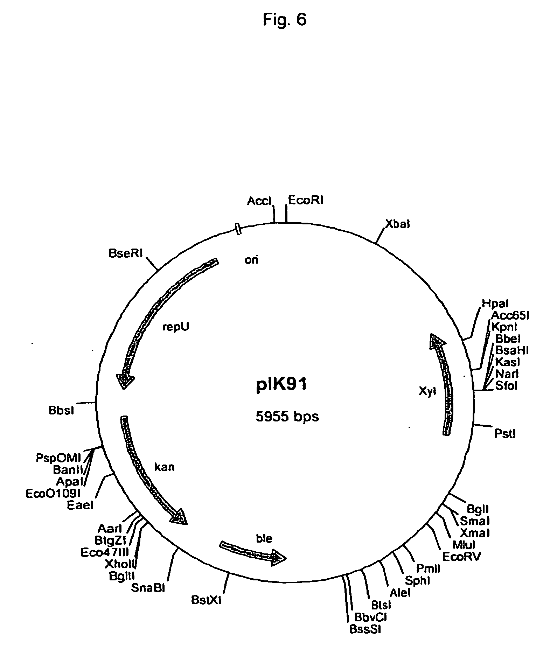

- Figur 6:: Plasmidkarte von pIK91
- Figur 7:: Plasmidkarte des zur Expression des Polypeptids verwendeten Vek- tors pTP15

Die Hinterlegung des Plasmids pTP01 aus Figur 4 und des Stammes RH 1626 erfolgte bei der DSMZ (Deutsche Sammlung von Mikroorganismen und Zellkulturen GmbH, Mascheroder Weg 1b, 38124 Braunschweig; http://www.dsmz.de) am 22.12.2006 bzw. am 18.12.2006 unter den Nummern DSM 18890 und DSM 18878. Bei *Bacillus amyloliquefaciens* RH 1626, der unter der Nummer DSM 18878 hinterlegt worden ist, handelt es sich um den Empfängerstamm mit einer *gpsA*-Deletion und ohne Plasmid. Die Hinterlegung DSM 18890 betrifft *Bacillus subtilis* RH 1632, der das Plasmid pTP01 trägt.

Die nachstehenden Beispiele erläutern die Erfindung näher.

### Beispiele

Die molekularbiologischen Arbeiten erfolgten nach Standardmethoden wie zum Beispiel im Handbuch von Sambrook und Russell (2001, Moleular cloning. Cold Spring Harbour Laboratory Press) beschrieben. Verwendete Baukästen (Kits) und Enzyme wurden nach Vorschrift des jeweiligen Herstellers eingesetzt.

### Beispiel 1: Isolierung des gpsA-Gens aus Bacillus amyloliquefaciens

Zur Isolierung des *gpsA-*Gens wurde chromosomale DNA aus *Bacillus amyloliquefaciens* (AB Enzymes GmbH Stammsammlung) mittels QIAGEN DNeasy Tissue Kit (Qiagen, Hilden) präpariert und hierauf das *gpsA*-Gen über PCR amplifiziert. Hierbei wurden Primer eingesetzt, die stromaufwärts und stromabwärts des *gpsA-*Gens hybridisieren, so dass das Gen vollständig amplifiziert wurde. Die Primer leiteten sich von den unter Beispiel 2 a) über Sequenzierung ermittelten Sequenzen der flankierenden Regionen von *gpsA* ab. Es wurden für die Amplifizierung folgende Primer verwendet:
Seq. ID No. 3: AL_1198 gctgttaagccgccgagcttcgttg
Seq ID No. 4: AR_180C taatcccatagcaccaagcgcaaaccac

Das 1591 bp enthaltene DNA-Fragment wurde vollständig über die Methode nach Sanger et al. (1977, Proc. Natl. Acad. Sci. USA 74, 5463-5467) sequenziert. Die verwendeten Sequenzier-Primer sind in Tabelle 1 aufgelistet.

**Tab. 1: Verwendete Primer für die vollständige Sequenzierung des gpsA-Gens aus Bacillus amyloliquefaciens.**

| Seq ID No. | Primer | Sequenz |
|---|---|---|
| 3 | AL_1198 | gctgttaagccgccgagcttcgttg |
| 4 | AR_180C | taatcccatagcaccaagcgcaaaccac |
| 5 | TPA fw 1 | cgacaaaagccattcgggaagtg |
| 6 | TPA fw 2 | tccgcgtctatacaaatcccg |
| 7 | TPA fw 3 | cgtaggcgatttaatcgtgac |

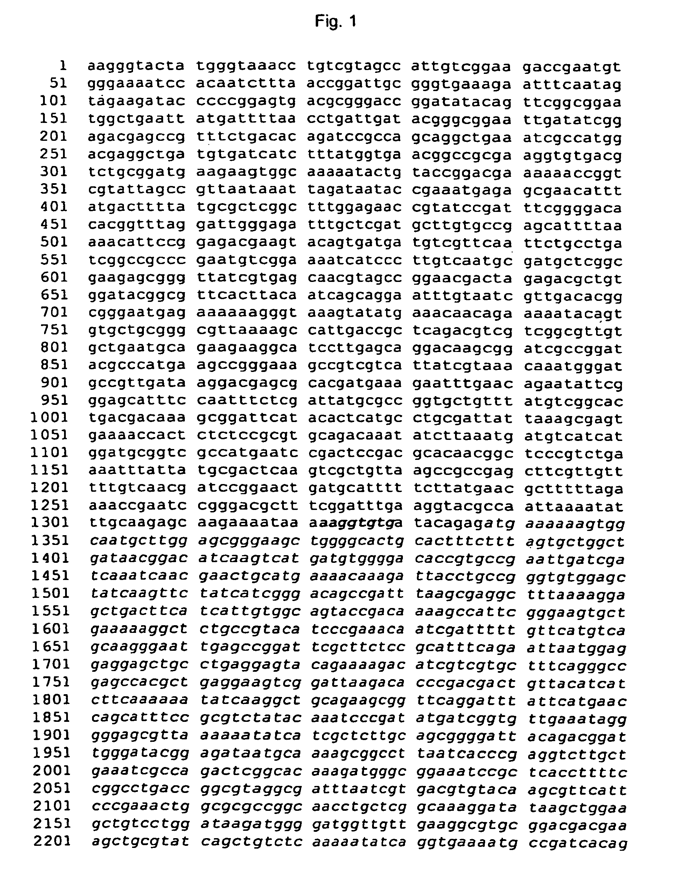

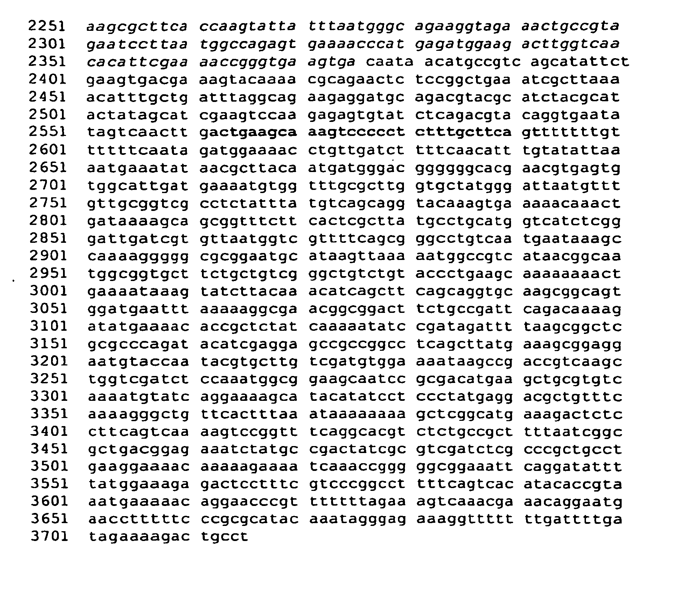

Das 1035 bp lange *gpsA*-Gen und die somit aus 345 Aminosäuren bestehende Glycerin-3-phosphatdehydrogenase ist mit den flankierenden DNA-Regionen in Figur 1 dargestellt.

### Beispiel 2: Deletion des gpsA-Gens in Bacillus amyloliquefaciens

Die Ausschaltung des Gens *gpsA* auf dem Chromosom von *B*. *amyloliquefaciens* wurde mittels eines Deletionsvektors vorgenommen. Die Vorgehensweise folgt der Beschreibung von Vehmaanperä et al. (1991). Als Vektor zur *gpsA*-Deletion wurde das in derselben Publikation beschriebene Plasmid pE194 ausgewählt. Der Vorteil dieses Vektors besteht darin, dass er einen Temperatur-abhängigen Replikationsursprung besitzt. Bei 28°C kann pE194 in der Zelle replizieren, so dass bei dieser Temperatur zunächst auf eine erfolgreiche Transformation selektiert werden kann. Anschließend werden die Zellen, die den Vektor enthalten, bei 46°C inkubiert. Bei dieser Temperatur repliziert der Vektor nicht mehr und es wird ein Selektionsdruck auf die Integration des Plasmids über einen der beiden homologen Bereiche (stromaufwärts oder stromabwärts von *gpsA*) in das Chromosom ausgeübt. Eine weitere homologe Rekombination über den anderen (zweiten) homologen Bereich führt dann zur *gpsA*-Deletion. Möglich wäre auch eine nochmalige Rekombination des ersten homologen Bereichs. Hierbei rekombiniert der Vektor wieder aus dem Chromosom heraus, so dass das chromosomale *gpsA*-Gen erhalten bliebe.

Die Elimination des *gpsA*-Gens aus dem Genom von *Bacillus amyloliquefaciens* RH 1330 umfasste folgende Schritte:
**Schritt 1: Konstruktion des Deletionsvektors**

### Isolation der gpsA flankierenden Regionen aus Bacillus amyloliquefaciens

Die chromosomale DNA aus *B*. *amyloliquefaciens* RH 1330 wurde nach einer Vorschrift von Sachse (In: Bertram und Gassen, 1991, Gentechnische Methoden. Gustav Fischer Verlag, Stuttgart, Jena, New York, 99-100) präpariert.

Die *gpsA* flankierenden Regionen von *B*. *amyloliquefaciens* RH 1330 wurden mittels PCR auf chromosomaler DNA amplifiziert. Da die Sequenzen dieser Regionen aus *B. amyloliquefaciens* nicht zur Verfügung standen, wurden verschiedene Primer aus der bekannten Sequenz *der yphC-gpsA-yphE-yphF*-Region aus *B*. *subtilis* (Datenbank SubtiList (Moszer et al., 1995; 1998) Stand: 18.02.2002) abgeleitet. Die Primer wurden in (in *B. subtilis*) nicht-codierende Bereiche gesetzt, um die Einführung von Mutationen in Genen innerhalb der flankierenden Regionen des *gpsA*-Gens zu vermeiden. Aufgrund der besonders niedrigen Sequenzidentität zwischen *B. amyloliquefaciens* und *B. subtilis* in diesen nicht-codierenden Bereichen erwies sich die Amplifizierung der das *gpsA*-Gen flankierenden Regionen durch PCR als schwierig und musste unter sehr milden Bedingungen (niedrige *annealing* Temperatur, hohe *template* Konzentration) durchgeführt werden.

Für die PCR Amplifizierung der stromaufwärts des *gpsA*-Gens liegenden Region wurden folgende Primer verwendet:

| | |
|---|---|
| Seq. ID No. 8: ALF.Xba.fw | aatgaaagcgtctagattgaaagg |
| Seq. ID No. 9: ALF.Kpn.bw | catgtttgattggtacctttttattttc |

Das PCR Produkt (ALF, 1.4kbp) wurde in das Plasmid pCR®2.1-TOPO® inseriert (Invitrogen, Carlsbad, US). Das entstandene Plasmid erhielt die Bezeichnung pCC1.

Für die PCR Amplifizierung der stromabwärts des *gpsA-Gens* liegenden Region wurden folgende Primer verwendet:

| | |
|---|---|
| Seq. ID No. 10: ARF.Kpn.fw | aagcgaaggtacccctctttg |
| Seq. ID No. 11: ARF.Xba.bw | cctatttgaatatgacatctctagaaaatttc |

Das PCR Produkt (ARF, 1.2kbp) wurde in das Plasmid pCR®2.1-TOPO® inseriert. Das entstandene Plasmid erhielt die Bezeichnung pCC2.

### Einbau der gpsA flankierenden Regionen aus Bacillus amyloliquefaciens in den Vektor pE194

Die ALF Region wurde aus dem mit *Kpn* I geschnittenen Plasmid pCC1 isoliert und in das mit dem gleichen Restriktionsenzym geschnittene Plasmid pCC2 inseriert. In dem entstandenen Plasmid pCC3 liegen die *gpsA* flankierenden Regionen *"head-to-tail"* vor.

Die *gpsA* flankierenden Regionen wurden durch Restriktion mit *Pst* I und *Sac* I aus dem Plasmid pCC3 isoliert und in den mit den gleichen Restriktionsenzymen geschnittenen Vektor pE194 eingebaut. Das entstandene Temperatur-abhängig replizierende Plasmid pCC10 ist der Deletionsvektor. Er wurde durch Restriktionsanalyse und Sequenzierung bestätigt.

### Schritt 2: Transformation von B. amyloliquefaciens mit dem Deletionsvektor und Deletion des gpsA-Gens im Chromosom

*B. amyloliquefaciens* RH 1330 wurde mit dem Deletionsvektor über Protoplasten nach der von Chang und Cohen (1979, Mol. Gen. Genet. 168, 111-115) beschriebenen Methode transformiert.

Danach erfolgte die Deletion des *gpsA*-Gens nach dem von Vehmaanperä et al. (1991) beschriebenen Verfahren. Der Deletionsvektor wurde zunächst unter Antibiotikum- und Temperaturdruck durch homologe Rekombination von einer der flankierenden Regionen des *gpsA*-Gens ins Chromosom integriert. Anschließend wurden die Zellen ohne Selektionsdruck durch Erythromycin gezüchtet, was eine zweite Rekombination zwischen beiden Kopien der zweiten homologen Region ermöglichte und zur Exzision des *gpsA* tragenden Deletionsvektors führte. Das chromosomal codierte *gpsA*-Gen wurde auf diese Weise vollständig aus dem *Bacillus* Genom entfernt.

Schließlich wurden die Zellen, bei denen die gewünschten Rekombinationsereignisse stattgefunden hatten, isoliert, indem die Zellen auf ihre Empfindlichkeit gegen Erythromycin und ihre Auxotrophie für Glycerin durch Wachstum auf entsprechenden Agarplatten untersucht wurden.

Der isolierte Stamm *B. amyloliquefaciens* RH 1330 A(*gpsA*) erhielt die Bezeichnung RH 1626. Die Abwesenheit des *gpsA*-Gens und die Erhaltung der flankierenden Regionen im Chromosom dieses Stammes sowie die Abwesenheit von Antibiotika-Resistenzgenen und anderen Sequenzen aus pE194 wurden durch Sequenzierung und Southern-Blot bestätigt.

### Beispiel 3: Konstruktion des rekombinanten Expressionssystems

Die Konstruktion des rekombinanten Expressionssystems umfasste folgende Schritte:

### Schritt 1: Insertion des gpsA-Gens in das pUB110-Derivat mit B. amyloliquefaciens Xylanase

Das *gpsA*-Gen wurde mit seiner eigenen ribosomalen Bindungsstelle (RBS) und seinem eigenen Transkriptions-Terminator ausgehend von chromosomaler DNA von *B. amyloliquefaciens* amplifiziert. Hierzu wurden folgende Primer verwendet:

| | |
|---|---|
| Seq ID No. 12: ABa Ale fw | cgaatccggcacgcttgtggatttg |
| Seq ID No. 13: ABa Sph bw | ccgtcccatcattgcatgcgttatatttc |

Die Primer wurden so konstruiert, dass stromaufwärts von der RBS eine Ale I-Schnittstelle und stromabwärts des Terminators eine Sph I-Schnittstelle in das PCR-Produkt inseriert wurden. Über diese eingeführten Schnittstellen konnte das erhaltene PCR-Fragment anschließend in den Xylanaseexpressionsvektor pIK91 (EP0585617) hinter dem von Zyprian und Matzura (1986) für pUB110 beschriebenen Promotor kloniert werden. Der Vektor pIK91, ein pUB110-Derivat (McKenzie et al., 1986, Plasmid 15, 93-103; McKenzie et al., 1987, Plasmid 17, 83-85), enthält das Gen für die endo-β-1,4-Xylanase (*xyl*) von *Bacillus subtilis,* reklassifiziert als *Bacillus amyloliquefaciens.* Das aus der Klonierung resultierende Plasmid pTP01 (dargestellt in Fig. 4) hat eine Größe von 7267 bp und trägt neben den Genen *gpsA* und *xyl* ein Kanamycin- und Bleomycin-Resistenzgen. Die Sequenz des entstandenen Plasmids pTP01 wurde durch Restriktionsanalyse und Sequenzierung bestätigt. Ein *Bacillus subtilis*-Stamm (1A 247, BGSC; Genotyp: *sacU(H), rpsL*) wurde mit pTP01 transformiert, wobei zur Transformation kompetente Zellen (Bertram und Gassen, 1991, Gentechnische Methoden. Gustav Fischer Verlag, Stuttgart, Jena, New York) verwendet wurden. Der resultierende Stamm erhielt die Bezeichnung RH 1632.

Die Expression von aktiver Glycerin-3-phosphatdehydrogenase konnte (entgegen früherer Berichte in der Literatur) im pTP01-tragenden Bacillus-Stamm RH 1632 über den von Morbidoni et al. (1995) und Bergmeyer (1970) beschriebenen Enzymaktivitätstest nachgewiesen werden. Bei diesem Aktivitätstest wird die Änderung der NADH-Konzentration während der von Glycerin-3-phosphatdehydrogenase katalysierten NADH-gekoppelten Umsetzung von Dihydroxyacetonphosphat zu sn-Glycerin-3-phosphat photometrisch verfolgt. Die Abnahme der Extinktion bei 340 nm ist der Konzentrationsabnahme des Substrates proportional. Der Bacillus-Stamm RH 1632 wurde hierbei im Schüttelkolben auf LB broth-Medium (1% Pepton, 0,5% Hefeextrakt, 1% NaCl, Leitungswasser, Einstellung des pH-Werts vor der Sterilisation auf pH 7,2) angezogen, die Zellen durch Zentrifugation gewonnen und durch Lysozym aufgeschlossen. Das Zelllysat wurde Für den Glycerin-3-phosphatdehydrogenase Aktivitätstest eingesetzt. Unter der Verwendung einer analogen Glycerin-3-phosphatdehydrogenase aus Kaninchenmuskel als Standard konnte gezeigt werden, dass der plasmidtragende Bacillus-Stamm RH 1632 im Vergleich zu *B*. *subtilis* 1A 247 eine um 2,0 Units g⁻¹ Zellen Feuchtgewicht höhere Aktivität hat. Bei der Transformante wird also zusätzlich zum chromosomal codierten *gpsA*-Gen das episomal codierte *gpsA*-Gen exprimiert.

Zur Überprüfung der Komplementierung des auxotrophen GpsA⁻ *Bacillus amyloliquefaciens*-Stammes RH 1626 (Beispiel 2) über pTP01 wurde der Stamm RH 1626 mit dem Plasmid pTP01 über Protoplasten (nach Chang und Cohen, 1979) transformiert. Die Selektion der Transformanten erfolgte über Antibiotikumselektion auf TYE-Agarplatten versetzt mit Xylan und Kanamycin (1% Pepton, 0,5% Hefeextrakt, 0,8% NaCl, 1 % Xylan, 10 g/l Kanamycin). Die Komplementierung des auxotrophen *B.amyloliquefaciens*-Stammes über pTP01 wurde über die Kultivierung auf Minimalmedium (Spizizen Salz (Anagnostopoulos und Spizizen, 1961, J. Bacteriol. 81(5), 741-746) mit 0,7% Glucose und 0,4% Glutamin, Wasser aufbereitet über das Millipore Water System, pH-Wert vor der Sterilisation bei pH 7,2) ohne Zugabe von Glycerin oder G3P nachgewiesen.

### Schritt 2: Deletion der Antibiotikamarkergene

Die Antibiotikaresistenzgene *kan* und *ble* wurden vom Plasmid pTP01 (Beispiel 3, Schritt 1) entfernt, indem das gesamte Plasmid ohne die Gene *kan* und *ble* durch PCR amplifiziert wurde. Es wurden hierbei Primer verwendet, die an jedem Ende des PCR-Produktes eine Sac II-Schnittstelle in das PCR-Produkt inserierten:

| | |
|---|---|
| Seq ID No. 14: pT ble kan Sac fw | gctaaaatctattattccgcggttcagcaatcgg |
| Seq ID No. 15: pT kan ble Sac bw | gtccattcactatccgcggtcccttttcag |

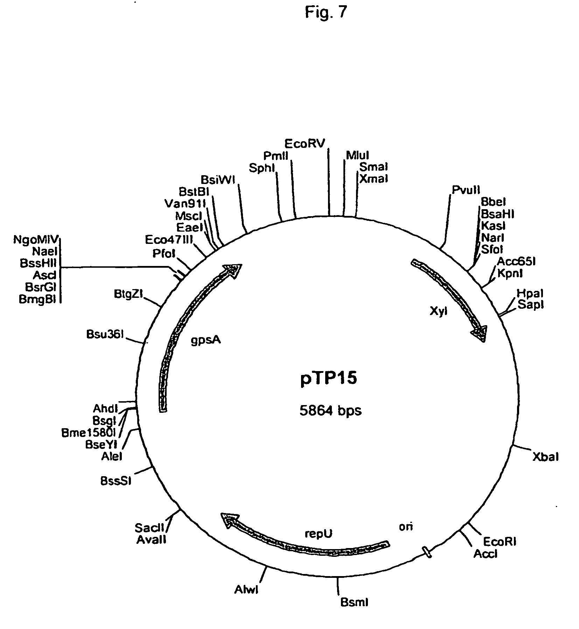

Das erhaltene PCR-Produkt wurde mit Sac II geschnitten, und das Restriktionsprodukt wurde religiert. Protoplasten des *B. subtilis*-Stammes BGSC 61106 (*gol* (= *gpsA) metC trpC2.* Morbidoni et al., 1995) wurden mit dem Ligationsprodukt transformiert. Der Stamm BGSC 61106 fungierte als Zwischenwirt. Eine direkte Transformation des Ligationsproduktes in den auxotrophen GpsA⁻ *B. amyloliquefaciens-*Stamm RH 1626 (Beispiel 2) war nicht möglich. Die Selektion der *Bacillus-*Transformanten erfolgte auf Agarplatten bestehend aus Spizizen Salz, 0,7% Glucose und 0,2% Glutamin. Das 5864 bp resultierende Plasmid wurde durch Kartierung und Sequenzierung bestätigt und erhielt die Bezeichnung pTP15 (Fig. 7).

### Schritt 3: Erstellung des rekombinanten Expressionssystems

Die Isolierung des Plasmids pT15 aus *B*. *subtilis* BGSC 61106 erfolgte über QIAGEN Plasmid MiniprepKit. Der *B*. *amyloliquefaciens*-Stamm RH 1626 (Δ*gpsA*; Beispiel 2) wurde über Protoplasten mit dem Plasmid pTP15 transformiert. Das resultierende Wirt-Vektorsystem, welches ohne das Vorhandensein von Antibiotikaresistenzgenen und ohne Antibiotika im Kulturmedium auskommt, wurde unter der Nummer RH 1810 in der AB Enzymes Stammsammlung registriert. Die Komplementierung des auxotrophen *B. amyloliquefaciens*-Stammes durch pTP15 wurde über die Kultivierung auf Minimalmedium (Spizizen Salz mit 0,7% Glucose und 0,4% Glutamin, Wasser aufbereitet über das Millipore Water System, pH-Wert vor der Sterilisation bei pH 7,2) nachgewiesen. RH 1810 wächst im Gegensatz zu RH 1626 auf Minimalmedium. Die Abwesenheit des *gpsA*-Gens auf der chromosomalen DNA von RH 1810 wurde über PCR durch Verwendung von Primern bewiesen, deren Sequenzen sich aus der flankierenden Region des *gpsA*-Gens ableiteten. Für den Nachweis wurden folgende Primer verwendet:

| | |
|---|---|
| Seq ID No. 16: A_313 | gaaggtgtgacgtctgcggatgaa |
| Seq ID No. 4: AR_180C | taatcccatagcaccaagcgcaaaccac |

Zur Überprüfung der Expression des xyl-Gens von RH 1810 wurde die Xylanaseaktivität im Kulturüberstand bestimmt. Dabei wurde der Bacillus-Stamm im Schüttelkolben zweistufig auf verschiedenen Medien gezüchtet. In der ersten Stufe erfolgte die Anzucht unter Selektionsdruck im Minimalmedium (Spizizen Salz mit 0,7% Glucose und 0,4% Glutamin, Wasser aufbereitet über das Millipore Water System, pH-Wert vor der Sterilisation bei pH 7,2). In der zweiten Stufe wurde Komplexmedium mit 5% der ersten Kultur beimpft. Das Medium entsprach folgender Zusammensetzung: 9% Glucidex 12, 2% Maisquellpulver, 1,32% (NH₄)₂HPO₄, 0,05% MgSO₄ * 7 H₂O, 0,5% CaCO₃, Leitungswasser, Einstellung des pH-Werts vor der Sterilisation auf pH 8,0. Die nach 48-stündiger Inkubation erhaltenen Kulturüberstände wurden zur Bestimmung der Xylanaseaktivität nach nachfolgender Methode verwendet.

Die durch enzymatische Spaltung von Xylan freigesetzten Xylanbruchstücke werden photometrisch bei 412 nm bestimmt. 1 Unit entspricht der Enzymmenge, die das Äquivalent von 1 µmol Xylose durch Spaltung aus Xylan in einer Minute bei 30°C unter Standardbedingungen freisetzt. Die Enzymverdünnungen werden mit 0,04 M Natriumacetatpufferlösung, pH 4,5 angesetzt. Der Reaktionsansatz für den Hauptwert besteht aus 0,75 ml einer 0,5%-igen Haferspelzenxylanlösung in 0,04 M Natriumacetatpuffer, pH 4,5 und 0,25 ml entsprechend verdünnter Enzymlösung. Beim Blindwert werden vor Zugabe der Enzymlösung 4 ml einer Lösung aus 0,5% p-Hydroxybenzoesäurehydrazid (PAHBAH, Firma Janssen Chimica), 0,465% Titriplex III (EDTA, Firma Merck) in 0,5 M NaOH zugegeben, um die Reaktion zu stoppen.

Nach der Inkubation von 20 min bei 30°C wird die enzymatische Reaktion im Hauptwert durch Zugabe von 4 ml der gleichen Lösung wie beim Blindwert gestoppt, und die Farbentwicklung durch Inkubation bei 75°C für 30 min durchgeführt. Hauptwert und Blindwert werden gegen Wasser im Photometer bei 412 nm gemessen. Die Auswertung erfolgt durch Kalibrierung mit einer Kalibrierkurve, bei der Xylose als Standard verwendet wird.

Bei der Doppelbestimmung der Xylanaseaktivitäten aus Kulturüberständen von RH 1810 wurden Aktivitäten von 153,4 XyIH g⁻¹ und 151,1 XyIH g⁻¹ gemessen. Die Antibiotikaresistenzgen-freie Xylanaseproduktion zeigte im Schüttelkolben somit eine höhere Produktivität als die Produktion mit RH 6000, bei der Kanamycin als Selektionsdruck verwendet wurde. Im Vergleich erzielte der rekombinante *Bacillus*-Stamm RH 6000 (RH 1330::pIK91) im Schüttelkolben eine Xylanaseaktivität von nur 57,8 XyIH g⁻¹ (EP 0585617 B1).

### Beispiel 4: Bestimmung der Stabilität des Systems

Zur Ermittlung der genetischen Stabilität des *gpsA*-xyl-tragenden Plasmids in den GpsA⁻ *B. amyloliquefaciens* Zellen wurde der nach Beispiel 3 erhaltene Stamm RH 1810 in einem Schüttelkolben-Experiment ohne Selektionsdruck in Flüssigmedium untersucht. Hierzu wurde eine Vorkultur von RH 1810 unter Selektionsdruck angezogen, d.h. dass ein Medium verwendet wurde, in dem der auxotrophe *B. amyloliquefaciens* nicht wachsen kann und in dem weder Glycerin oder G3P noch Edukte von Glycerin oder G3P für den *Bacillus*-Stamm zugänglich sind. Die Anzucht erfolgte im 150 ml-Erlenmeyer-Schüttelkolben mit jeweils 20 ml Medium. Das Medium der Vorkultur bestand aus folgender Zusammensetzung: Spizizen Salz + 7% Glucidex 12 + 2% Caseinhydrolysat (pH-Wert bei pH 7,2). Die erste Vorkultur wurde über 16 h inkubiert und aus dieser die zweite Vorkultur mit gleicher Medienzusammensetzung beimpft. Nach 8-stündiger Inkubation wurde hieraus die Hauptkultur beimpft. Die Anzucht ohne Selektionsdruck erfolgte im 11-Erlenmeyer-Schüttelkolben mit jeweils 150 ml Medium der Zusammensetzung: 9% Glucidex 12, 2% Maisquellpulver, 1,32% (NH₄)₂HPO₄, 0,05% MgSO₄ * 7 H₂O, 0,5% CaCO₃, Leitungswasser, Einstellung des pH-Werts vor der Sterilisation auf pH 8,0. In diesem Medium ist ein Wachstum des GpsA⁻ *Bacillus*-Stammes ohne das komplementierende Plasmid pTP15 (Beispiel 3: Schritt 2) möglich. Nach 8 bis 16 Stunden wurde die Kultur mit 5% in jeweils zwei Erlenmeyer-Schüttelkolben mit frischem Medium überimpft, wobei der eine Kolben zur Überimpfung des Folgekolbens diente, und aus dem anderen nach 24-stündiger Kultivierung die Xylanaseaktivität des Kulturüberstands ermittelt wurde. Die Kultivierung erfolgte über fünf Tage und Nächte; hierbei wurden die Hauptkulturen viermal überimpft. Nach jeder Beendigung der Kultivierung wurde die Gesamtzellzahl pro ml Medium bestimmt, um die Plasmidstabilität von pT15 in RH 1810 über die Generationszahl überprüfen zu können. Das Ergebnis ist in Tabelle 2 dargestellt.

**Tab. 2: Plasmidstabilität in den pTP15-tragenden Transformanten von GpsA⁻ Bacillus amyloliquefaciens Zellen RH 1626, nachgewiesen anhand der Xylanaseaktivität über die Generationszahl.**

| RH 1810 : *Bacillus amyloliquefaciens* Δ(gpsA)::pTP15 | | | |
|---|---|---|---|
| Akkumulierte Kultivierungsdauer der Hauptkultur [h] | Überimpfungsanzahlder Hauptkultur | Generationszahl | Relative Xylanaseaktivität [%] |
| 24 | 1 | 8 | 100 |
| 40 | 2 | 12 | 96 |
| 48 | 3 | 16 | 97 |
| 64 | 4 | 21 | 111 |
| 72 | 5 | 25 | 111 |

Wie das Ergebnis in Tabelle 2 zeigt, bleibt die Xylanaseaktivität ohne Selektionsdruck über die 5-fache Dauer einer normalen Fermentation stabil. Dies zeigt sich daran, dass die relative Xylanaseaktivität in Bezug auf die erste Kultur ohne Selektionsdruck, konstant bleibt und sogar leicht weiter ansteigt.

### Beispiel 5: Herstellung von Xylanase durch Fermentation in einem Bioreaktor mit dem rekombinanten System

### a) Vorkultur

Der Stamm RH 1810 wurde auf Selektionsplatten (Spizizen Salz mit 0,7% Glucose, 0,4% Glutamin und 1% Agar, Wasser aufbereitet über das Millipore Water System, pH-Wert vor der Sterilisation 7,2) angezogen. Die Inkubation erfolgte bei 37°C für mindestens 32 h.
1. Vorkultur: Ein 1 I-Erlenmeyerkolben mit Schikanen mit 150 ml Medium wurde mit RH 1810 von der Agarplatte beimpft. Die Nährlösung hatte folgende Zusammensetzung:

| | |
|---|---|
| (NH₄)₂SO₄ | 2 g l⁻¹ |
| K₂HPO₄ | 14 g l⁻¹ |
| KH₂PO₄ | 6 g l⁻¹ |
| Na₃citrat x 2 H₂O | 1g l⁻¹ |
| M_{g}SO₄ x 7 H₂O | 0,2 g l⁻¹ |
| Glucidex 12 | 70 g l⁻¹ |
| Caseinhydrolysat | 20 g l⁻¹ |
| pH 7,2; Sterilisation 30 min 121°C | |

Die Kultur wurde für 16 Stunden bei 37°C unter Schütteln (150 Upm) inkubiert.
2. Vorkultur: 1 1-Erlenmeyerkolben mit Schikanen wurden mit 150 ml Medium gleicher Zusammensetzung gefüllt und mit 5% der 1. Vorkultur beimpft. Die Kultivierung erfolgt bei 37°C für 8 Stunden.

### b) Bioreaktor

Hauptkultur: 20 1 einer Nährlösung aus

| | |
|---|---|
| Glucidex 12 | 90 g l⁻¹ |
| Maisquellpulver | 20 g l⁻¹ |
| MgSO₄ x 7 H₂O | 0,5 g l⁻¹ |
| CaCO₃ | 5 g l⁻¹ |
| (NH₄)₂HPO₄ | 13,2 g l⁻¹ |
| pH = 7,2; Sterilisation 30 min 121°C | |

Der Bioreaktor wird mit 5 % der 2. Vorkultur beimpft. Kulturbedingungen: 37°C, Belüftung 0,5 vvm, Rühren mit 450 Upm, 48 Stunden. Die Kulturbrühe wurde durch Zentrifugation geklärt und zur Xylanaseaktivitätsbestimmung verwendet. Im Kulturüberstand konnte eine Xylanaseaktivität von 166 XyIH g⁻¹ gemessen werden.

### c) Backversuch

Aus 100 Gewichtsteilen Weizenmehl, 2 Gewichtsteilen Salz, 3 Gewichtsteilen Backhefe, 58 - 60 Gewichtsteilen Wasser und 40-50 ppm (bez. Teiggewicht) Ascorbinsäure wurde in einem Spiralkneter (Fabrikat Diosna) 2-3 min auf niedriger Stufe I und 3-4 min auf höherer Stufe II ein Teig bereitet. Dem Wasser wurden vor Beginn des Knetvorganges die entsprechenden Enzymmengen zugesetzt. Die Teigtemperatur betrug 25°-28°C. Nach einer Teigruhe von 10 min wurde der Teig zur Herstellung von freigeschobenem Weißbrot in 350 g-Teigstücke geteilt. Nach einer weiteren Teigruhe von 20 min wurden die 350 g-Teigstücke geformt, 70 min bei 32°C und 80% relativer Luftfeuchte gegart und 32 min bei 230°C gebacken.

Das Volumen der Brote wurde nach deren Auskühlen mit Hilfe eines TexVol Instruments BVM-L370 per Laserabtastung vermessen. Der Mittelwert aller vier Brote aus einem Teigansatz wird bei der Auswertung berücksichtigt.

### Ergebnis der Backversuche

Für Backversuche wurde Kulturüberstand einer Fermentation von RH 1810, die nach den im Beispiel 5 a) und b) beschriebenen Bedingungen durchgeführt wurde, eingesetzt. Brote, die nach der erläuterten Rezeptur mit dem erfindungsgemäß hergestellten Enzym gebacken wurden, zeigten folgende Eigenschaften:

**Tab 3.: Konzentrationsreihe mit der erfindungsgemäß hergestellten backaktiven Xylanase aus RH 1810 im Vergleich zur Xylanase, produziert mit dem rekombinanten Bacillus-Stamm RH 6000 (EP 0585617 B1). Das Volumen der Brote, denen die backaktive Xylanase aus RH 6000 zugesetzt wurde, ist 100%.**

| Dosage UXyl je 100 kg Mehl | Volumen [%] |
|---|---|
| 1800 | 101 |
| 3600 | 102 |
| 5400 | 99 |

## Patentansprüche

1. Expressionssystem zur Produktion eines oder mehrerer Zielpolypeptids/Zielpolypeptide, umfassend eine Wirtszelle, in deren Genom die DNA-Sequenz, die Glycerin-3-phoshatdehydrogenase codiert, inaktiviert oder teilweise oder vollständig deletiert ist wobei die Deletion die Inaktivierung des glycerin-3-phosphatdehydrogenasegens bewirkst und die mit einem extrachromosomalen Element, das eine DNA-Sequenz, die das bzw. die Zielpolypeptid(e) und Glycerin-3-phosphatdehydrogenase codiert, umfasst, transformiert worden ist, wobei sowohl das Wirtszellgenom als auch das extrachromosomale Element kein Antibiotikaresistenzgen tragen.

2. Expressionssystem nach Anspruch 1, **dadurch gekennzeichnet, dass** das extrachromosomale Element ein Plasmid, ein Phage, ein Phagmid oder ein Transposon ist.

3. Expressionssystem nach einem der Ansprüche 1 oder 2, **dadurch gekennzeichnet, dass** das extrachromosomale Element eine DNA-Sequenz, die der die endogene Glycerin-3-phosphatdehydrogenase der Wirtszelle codierenden DNA-Sequenz entspricht, eine DNA-Sequenz, die eine verwandte Glycerin-3-phosphatdehydrogenase codiert oder eine fremde Glycerin-3-phosphatdehydrogenase codiert, umfasst.

4. Expressionssystem nach einem der Ansprüche 1 bis 3, **dadurch gekennzeichnet, dass** die Glycerin-3-phosphatdehydrogenase codierende DNA-Sequenz unter der Kontrolle eines schwachen Promotors steht.

5. Expressionssystem nach Anspruch 4, **dadurch gekennzeichnet, dass** der schwache Promotor der in Fig. 7 stromaufwärts des gpsA-Gens liegende Promotor aus dem Plasmid pUB110 ist.

6. Expressionssystem nach Anspruch 4, **dadurch gekennzeichnet, dass** der schwache Promotor der ptsH-Promotor aus *B. subtilis* ist.

7. Expressionssystem nach einem der Ansprüche 1 bis 6, **dadurch gekennzeichnet, dass** die endogene Glycerin-3-phosphatdehydrogenase codierende DNA-Sequenz im Wesentlichen punktgenau und ohne Mutation oder Leserasterverschiebung in den angrenzenden Bereichen deletiert ist.

8. Expressionssystem nach einem der Ansprüche 1 bis 7, **dadurch gekennzeichnet, dass** die Wirtszelle von einer gram-positiven Bakterienzelle abgeleitet ist.

9. Expressionssystem nach Anspruch 8, **dadurch gekennzeichnet, dass** die Wirtszelle von einer Zelle der Gattung *Staphylococcus, Corynebacterium* oder *Bacillus* abgeleitet ist.

10. Expressionssystem nach Anspruch 9, **dadurch gekennzeichnet, dass** die Wirtszelle von *Bacillus amyloliquefaciens* abgeleitet ist und bevorzugt *Bacillus amyloliquefaciens* RH 1626, hinterlegt unter der Hinterlegungsnummer DSM 18878, ist.

11. Expressionssystem nach einem der Ansprüche 1 bis 7, **dadurch gekennzeichnet, dass** die Wirtszelle von einer gram-negativen Bakterienzelle abgeleitet ist.

12. Expressionssystem nach einem der Ansprüche 1 bis 12, **dadurch gekennzeichnet, dass** das Zielpeptid ausgewählt ist aus hydrolytischen Enzymen, Pektin-abbauenden Enzymen, proteolytischen Enzymen oder lipolytischen Enzymen.

13. Expressionssystem nach einem der Ansprüche 1 bis 13, **dadurch gekennzeichnet, dass** die DNA-Sequenz, die Glycerin-3-phosphatdehydrogenase codiert, ausgewählt ist aus
a) einer Nucleotidsequenz gemäß SEQ ID NO:1,
b) einer DNA-Sequenz, die für eine Proteinsequenz gemäß SEQ ID NO: 2 codiert,
c) einer DNA-Sequenz, die unter stringenten Bedingungen mit einer Sequenz gemäß a) oder b) hybridisiert, oder
d) einer DNA-Sequenz, die aufgrund der Degeneriertheit des genetischen Codes mit den Sequenzen gemäß a), b), oder c) verwandt ist, oder
e) komplementären Strängen zu den Sequenzen a) bis d).

14. Verfahren zur antibiotikafreien Produktion eines Zielpolypeptids, **dadurch gekennzeichnet, dass** man ein Expressionssystem nach einem der Ansprüche 1 bis 14 unter Bedingungen zur Expression des Zielpolypeptids züchtet und das so exprimierte Zielpolypeptid isoliert.

15. Verwendung eines Expressionssystems nach einem der Ansprüche 1 bis 14 zur antibiotikafreien Produktion eines Zielpolypeptids.

## Claims

1. An expression system for producing one or more target polypeptide(s), comprising a host cell in the genome of which the DNA sequence encoding the glycerol-3-phosphate dehydrogenase is inactivated or partially or completely deleted, such deletion causing inactivation of the glycerol-3-phosphate dehydrogenase gene, and which has been transformed with an extrachromosomal element comprising a DNA sequence encoding the target polypeptide(s) and glycerol-3-phosphate dehydrogenase, both the host cell genome and the extrachromosomal element bearing no antibiotic resistance gene.

2. The expression system according to claim 1, **characterised in that** the extrachromosomal element is a plasmid, a phage, a phagemid or a transposon.

3. The expression system according to one of the claims 1 or 2, **characterised in that** the extrachromosomal element comprises a DNA sequence corresponding to the DNA sequence encoding the endogenous glycerol-3-phosphate dehydrogenase of the host cell, a DNA sequence encoding a related glycerol-3-phosphate dehydrogenase or a foreign glycerol-3-phosphate dehydrogenase.

4. The expression system according to any of the claims 1 to 3, **characterised in that** the DNA sequence encoding the glycerol-3-phosphate dehydrogenase under the control of a weak promoter.

5. The expression system according to claim 4, **characterised in that** the weak promoter is the promoter from the plasmid pUB110 located upstream of the gpsA gene in Fig. 7.

6. The expression system according to claim 4, **characterised in that** the weak promoter is the ptsH promoter from *B. subtilis.*

7. The expression system according to any of the claims 1 to 6, **characterised in that** the endogenous DNA sequence encoding glycerol-3-phosphate dehydrogenase is deleted substantially to the point and without mutation or frame shift in the adjacent regions.

8. The expression system according to any of the claims 1 to 7, **characterised in that** the host cell is derived from a Gram-positive bacterial cell.

9. The expression system according to claim 8, **characterised in that** the host cell is derived from a cell of the species *Staphylococcus, Corynebacterium* or *Bacillus.*

10. The expression system according to claim 9, **characterised in that** the host cell is derived from *Bacillus amyloliquefaciens* and preferably *Bacillus amyloliquefaciens* RH 1626, deposited under accession number DSM 18878.

11. The expression system according to any of the claims 1 to 7 , **characterised in that** the host cell is derived from a Gram-negative bacterial cell.

12. The expression system according to any of the claims 1 to 11, **characterised in that** the target peptide is selected from hydrolytic enzymes, pectin-degrading enzymes, proteolytic enzymes or lipolytic enzymes.

13. The expression system according to any of the claims 1 to 13, **characterised in that** the DNA sequence encoding the glycerol-3-phosphate dehydrogenase is selected from:
a) a nucleotide sequence according to SEQ ID NO: 1,
b) a DNA sequence encoding for a protein sequence according to SEQ ID NO: 2,
c) a DNA s-equence hybridising with a sequence according to a) or b) under stringent conditions, or
d) a DNA sequence related to the sequences according to a), b) or c) as a result of the degeneration of genetic code, or
e) strands complementary to sequences a) to d).

14. A method for the production of a target polypeptide free of antibiotics, **characterised in that** an expression system according to any of the claims 1 to 14 is cultivated under conditions suitable for expressing the target polypeptide and the target polypeptide thus expressed is isolated.

15. The use of an expression system according to any of the claims 1 to 14 for producing a target polypeptide free of antibiotics.

## Revendications

1. Système d'expression pour la production d'un polypeptide cible/des plusieurs polypeptides cibles comprenant une cellule hôte, dans le génome de celle-ci la séquence d'ADN, codante de la glycéro-3-phosphate déshydrogénase est inactivée ou délétée complètement ou partiellement, la délétion provoquant l'inactivation du gène de la glycëro-3-phosphate déshydrogénase, et qui est transformée par un élément extrachromosomique qui comprend une séquence d'ADN codante du polypeptide cible/des polypeptides cibles et de la glycéro-3-phosphate déshydrogénase ; dans lequel le génome de la cellule hôte et l'élément extrachromosomique ne portent pas un gène de la résistance aux antibiotiques.

2. Système d'expression selon la revendication 1, **caractérisé en ce que** l'élément extrachromosomique est un plasmide, un phage ou un transposon.

3. Système d'expression selon l'une quelconque des revendications 1 et 2, **caractérisé en ce que** l'élément extrachromosomique comprend une séquence correspondante de la séquence d'ADN codante de la glycéro-3-phosphate déshydrogénase endogène de la cellule hôte, une séquence d'ADN codante d'une glycéro-3-phosphate déshydrogénase apparentée ou d'une glycéro-3-phosphate déshydrogénase étrangère.

4. Système d'expression selon l'une quelconque des revendications 1 à 3, **caractérisé en ce que** la séquence d'ADN codante de la glycéro-3-phosphate déshydrogénase est sous le contrôle d'un promoteur faible.

5. Système d'expression selon la revendication 4, **caractérisé en ce que** le promoteur faible est le promoteur qui est situé dans la figure 7 en amont du gène gpsA et qui est dérivé du plasmide pUB110.

6. Système d'expression selon la revendication 4, **caractérisé en ce que** le promoteur faible est le promoteur ptsH de B. subtilis.

7. Système d'expression selon l'une quelconque des revendications 1 à 6, **caractérisé en ce que** la séquence d'ADN codante de la glycéro-3-phosphate déshydrogénase endogène est délétée essentiellement par une mutation ponctuelle et sans une mutation ou une déplacement du cadre de la lecture dans les régions voisines.

8. Système d'expression selon l'une quelconque des revendications 1 à 7, **caractérisé en ce que** la cellule hôte est dérivée d'une cellule de bactérie Gram positive.

9. Système d'expression la revendication 8, **caractérisé en ce que** la cellule hôte est dérivée d'une cellule de l'espèce de Staphylococcus, Corynebacterium ou Bacillus.

10. Système d'expression la revendication 9, **caractérisé en ce que** la cellule hôte est dérivée de Bacillus amyloliquefaciens et particulièrement Bacillus amyloliquefaciens RH 1626, déposé sous le numéro DSM 18878.

11. Système d'expression selon l'une quelconque des revendications 1 à 7, **caractérisé en ce que** la cellule hôte est dérivée d'une cellule bactérie Gram-négative.

12. Système d'expression selon l'une quelconque des revendications 1 à 12, **caractérisé en ce que** le peptide cible est choisi parmi les enzymes hydrolytiques, les enzymes dégradant la pectine, les enzymes protéolytiques ou les enzymes lipolytiques.

13. Système d'expression selon l'une quelconque des revendications 1 à 13, **caractérisé en ce que** la séquence d'ADN codante de la glycéro-3-phosphate déshydrogénase est choisie parmi
a) une séquence d'ADN selon SEQ ID NO :1,
b) une séquence d'ADN codante d'une séquence de protéine selon SEQ ID NO :2
c) une séquence d'ADN qui hybride dans des conditions stringentes à une séquence selon a) ou b), ou
d) une séquence d'ADN qui est apparantée aux séquences selon a), b) ou c) du fait de la dégénérence du code génetique, ou
e) des brins complémentaires aux séquences a) à d).

14. Procédé pour la production d'un polypeptide cible sans antibiotique **caractérisé en ce qu'**on cultive un système d'expression selon l'une quelconque des revendications 1 à 14 dans des conditions pour l'expression du peptide cible et on isole le polypeptide exprimé.

15. Utilisation d'un système d'expression selon l'une quelconque des revendications 1 à 14 pour la production d'un polypeptide cible sans antibiotique.
